(19) Europäisches Patentamt
European Patent Office
Office européen des brevets

(11) EP 2 069 334 B2

(12) # NEUE EUROPÄISCHE PATENTSCHRIFT

Nach dem Einspruchsverfahren

(45) Veröffentlichungstag und Bekanntmachung des Hinweises auf die Entscheidung über den Einspruch:
**05.11.2014 Patentblatt 2014/45**

(45) Hinweis auf die Patenterteilung:
**16.11.2011 Patentblatt 2011/46**

(21) Anmeldenummer: **08801873.4**

(22) Anmeldetag: **05.09.2008**

(51) Int Cl.:
***C07D 407/10*** $^{(2006.01)}$ ***A61K 31/353*** $^{(2006.01)}$
***A61P 25/00*** $^{(2006.01)}$

(86) Internationale Anmeldenummer:
**PCT/EP2008/007279**

(87) Internationale Veröffentlichungsnummer:
**WO 2009/052895 (30.04.2009 Gazette 2009/18)**

(54) **ASPALATHIN-ÄHNLICHES DIHYDROCHALCON, EXTRAKTE AUS UNFERMENTIERTEM ROTBUSCH UND VERFAHREN ZUR HERSTELLUNG**

ASPALATHIN-LIKE DIHYDROCHALCONE, EXTRACTS FROM UNFERMENTED ROOIBOS AND PROCESS FOR PREPARATION

DIHYDROCHALCONE DU TYPE ASPALATHINE, EXTRAIT PROVENANT DU ROOIBOS NON FERMENTÉ ET SON PROCÉDÉ DE PRODUCTION

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **23.10.2007 EP 07020728**

(43) Veröffentlichungstag der Anmeldung:
**17.06.2009 Patentblatt 2009/25**

(73) Patentinhaber: **Kneipp GmbH**
**97084 Würzburg (DE)**

(72) Erfinder:
- **FRANK, Bruno**
  **97271 Kleinrinderfeld (DE)**
- **DIMPFEL, Wilfried**
  **35578 Wetzlar (DE)**

(74) Vertreter: **Keller, Günter et al**
**Lederer & Keller**
**Patentanwälte Partnerschaft mbB**
**Unsöldstrasse 2**
**80538 München (DE)**

(56) Entgegenhaltungen:
**WO-A1-2008/110551 DE-A1-102005 004 438**
**DE-A1-102005 004 438 JP-A- H11 116 429**

- **H. SCHULZ ET AL.: "Quantification of quality parameters for reliable evaluation of green rooibos (Aspalathus linearis)" EUR. FOOD RES. TECHNOL., Bd. 216, 2003, Seiten 539-543, XP002462520 in der Anmeldung erwähnt**



Printed by Jouve, 75001 PARIS (FR)

## Beschreibung

Die vorliegende Erfindung betrifft eine neue chemische Verbindung der Formel I sowie deren pharmazeutisch annehmbare Salze, Derivate und Ester. Zudem betrifft die Erfindung auch ein Verfahren zur Isolierung der Verbindung gemäß Formel I aus Rotbusch-Rohware. Die vorliegende Erfindung betrifft ebenfalls einen Rotbuschextrakt, der einen Gehalt an der Verbindung gemäß Formel I von mindestens 0,4 Gew.-%, bevorzugt mindestens 1,5 Gew.-% aufweist. Desweiteren betrifft die Erfindung die Verwendung der chemischen Verbindung der Formel I sowie deren pharmazeutisch annehmbare Salze, Derivate und Ester sowie des erfindungsgemäßen Rotbuschextrakts als Medikament, insbesondere zur Vorbeugung und Behandlung von neurologischen und psychiatrischen Störungen des zentralen Nervensystems, insbesondere Demenzen. Der Ausdruck pharmazeutisch wirksam umfasst auch solche Wirkungen, die zu einer subjektiven Verbesserung der Befindlichkeit führen, wobei eine arzneimittelrechtliche Zulassung nicht unbedingt erforderlich sein muss.

Der Rotbusch (englisch: Rooibos; lateinisch: Aspalathus linearis) wächst ausschließlich in Südafrika und enthält derzeit als weltweit einzige bekannte Pflanze die besonders stark antioxidativ wirkende Substanz Aspalathin, ein Flavonoid. Zudem enthält der Rotbusch weitere Flavonoide, wie C-Glycosylflavone (unter anderem Orientin, Isoorientin), Flavonol-3-O-Glycoside (unter anderem Quercetin, Quercitrin, Isoquercitrin, Rutin) und die Dihydrochalcone Nothofagin und Aspalathin.

Unfermentierter "grüner" Rotbusch zeichnet sich durch einen höheren Gehalt an Polyphenolen, insbesondere Aspalathin, sowie einer höheren antioxidativen Aktivität im Vergleich zum fermentierten Produkt aus. Der Effekt der Abnahme der antioxidativen Aktivität durch den Fermentationsprozess ist gleichermaßen beim schwarzen gegenüber dem grünen Tee zu beobachten (Bramati et al., J. Agric. Food Chem. 2003, 51: 7472-7474). Wissenschaftliche Untersuchungen haben gezeigt, dass die antioxidative Aktivität des Rotbuschtees hauptsächlich auf den Aspalathingehalt zurückzuführen ist. Bei der Untersuchung des Fermentationsprozesses von Rotbuschtee wurde festgestellt, dass der Gehalt an Aspalathin und Nothofagin während des Fermentationsprozesses abnimmt (Schulz et al., Eur. Food Res. Technol. 2003, 216: 539-543). Die geringere antioxidative Aktivität von fermentiertem "rotem" Rotbuschtee gegenüber unfermentiertem "grünem" Rotbuschtee kann somit erklärt werden.

Aufgrund der vorstehend genannten gesundheitsfördernden Flavonoide sowie seinem guten Geschmack ist der Rotbuschtee weit verbreitet. Weitere Inhaltsstoffe des Rotbuschtees sind Phenolsäuren, ätherisches Öl, Vitamin C sowie zahlreiche Mineralstoffe, insbesondere Eisen und Fluorid.

Um eine möglichst hohe antioxidative Aktivität zu erzielen, ist ein hoher Gehalt an Aspalathin notwendig. Diesbezüglich offenbart DE 10 2005 004 438 einen Rotbuschextrakt, der im Vergleich zu dem üblichen Aspalathingehalt von 1 bis 3 Gew.-% einen erhöhten Gehalt von mehr als 5 Gew.-%, bei gleichzeitig niedrigem Chlorophyllgehalt von weniger als 0,4 Gew.-%, aufweist. Gemäß DE 10 2005 004 438 wird der Rotbuschextrakt durch Extraktion aus unfermentierter Rotbusch-Rohware unter Verwendung eines Gemischs aus Ethanol und Wasser erhalten, wobei eine Ethanol/Wassermischung im Verhältnis 80 zu 20 verwendet wird.. Der Rotbuschextrakt mit hohem Aspalathingehalt soll aufgrund der stark antioxidativen, antiirritativen und antimikrobiellen Wirkung insbesondere für die kosmetische Anwendung beispielsweise als Haar-, Haut- oder Mundpflegemittel verwendet werden.

Ein weiteres Flavonoid, das in dem Rotbuschtee enthalten ist, ist Quercetin. Dies kommt mit einem Gehalt von ca. 11 mg/100 g Rotbusch-Rohmaterial vor und wirkt sich z.B. auf die Histaminfreisetzung im menschlichen Körper aus, wodurch allergische Symptome gelindert werden können. Quercetin vermag ebenso die Produktion der Monoaminooxidase zu hemmen, was leichte Depressionen und Einschlafstörungen günstig beeinflusst (Plantextrakt, the nature network, Ausgabe 3 vom 09.11.2005; Plantextrakt GmbH).

Ein Ausgangspunkt der vorliegenden Erfindung war die Suche nach Wirkstoffen zur Behandlung von Krankheiten des zentralen Nervensystems, wie beispielsweise Demenzen, Morbus Parkinson, Depressionen und Schmerzzustände. Diese Krankheiten sind schwer zu therapieren, und die hierbei eingesetzten Medikamente wie beispielsweise Tacrin, Galantamin oder Nefopam weisen ein breites Nebenwirkungsspektrum auf.

Eine Aufgabe der Erfindung ist es daher, Wirkstoffe, Zusammensetzungen sowie Extrakte aus Rotbusch zur therapeutischen Behandlung dieser Krankheiten zur Verfügung zu stellen. Insbesondere sollen diese Wirkstoffe oder Zusammensetzungen geringe Nebenwirkungen aufweisen.

Diese Aufgabe wird durch den Gegenstand der Patentansprüche gelöst.

Die vorliegende Erfindung betrifft eine Verbindung der Formel I

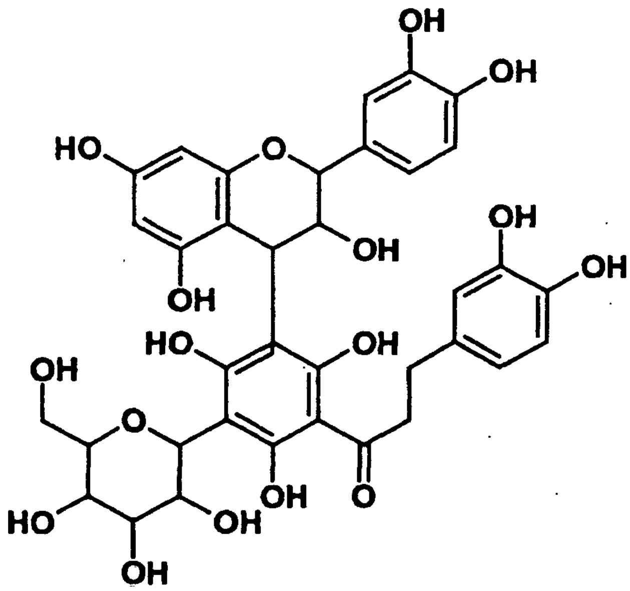

sowie deren pharmazeutisch annehmbare Salze, Derivate und Ester. Bevorzugte Derivate sind hierbei Kopplungsprodukte der Verbindung gemäß Formel I an Ferulasäure, Chinasäure, Kaffeesäure, Gluconsäure oder Chlorogensäure. Bevorzugt wird die Verbindung der Formel I in ihrer natürlichen Form oder als Salz eingesetzt, weiter bevorzugt in ihrer natürlichen Form gemäß Formel I.

Unter den bevorzugten Estern sind Ameisensäure-, Essigsäure-, Propionsäure-, Glutarsäure-, Weinsäure- oder Bernsteinsäureester zu nennen. Bevorzugte Salze sind die Salze mit kationischen, organischen oder anorganischen Gegenionen zu nennen, insbesondere Alkalimetallsalze, Erdalkalimetallsalze, Ammoniumsalze oder auch Salze mit pharmazeutisch annehmbaren Säuren wie Succinate, Citrate, Tartrate.

Desweiteren betrifft die Erfindung einen Rotbuschextrakt, aus unfermentiertem Rotbusch, mit einem Gehalt an der Verbindung gemäß Formel I von mindestens 0,4 Gew.-%, weiter bevorzugt mindestens 1,5 Gew.-%, weiter bevorzugt mindestens 2,5 Gew.-%, weiter besonders bevorzugt mindestens 5 Gew.-%, weiter noch bevorzugter mindestens 10 Gew.-% und am meisten bevorzugt mindestens 20 Gew.-%.

Desweiteren betrifft die Erfindung ein Verfahren zur Herstellung der Verbindung der Formel I, bei dem

- getrocknete und zerkleinerte, unfermentierte Rotbusch-Rohware mit einem Extraktionsmittel bestehend aus Methanol und/oder Wasser für eine vorbestimmte Extraktionsdauer bei einer Temperatur von bis zu 90°C, bevorzugt bis zu 60°C extrahiert wird,
- das Extrakt filtriert und anschließend unter reduziertem Druck zur Trockne eingeengt wird, und anschließend
- durch mehrere, bevorzugt drei chromatographische Trennungsschritte unter Verwendung von zwei Sephadex LH20-Säulen und anschließend einer lipophilen c18-HPLC-Säule aufgereinigt wird.

Die Erfindung betrifft auch die Verwendung eines Rotbuschextrakts als Medikament oder Nahrungsergänzungsmittel. Die Erfindung betrifft zudem die Verwendung von Verbindungen gemäß Formel I sowie deren pharmazeutisch annehmbare Salze, Derivate und Ester als Medikament oder Nahrungsergänzungsmittel. Das Nahrungsergänzungsmittel oder Medikament kann den Rotbuschextrakt beispielsweise in einer Menge von mindestens 10 mg, mindestens 20 mg oder mindestens 50 mg pro g Nahrungsergänzungsmittel bzw. pro Dosierungseinheit . des Medikaments enthalten. Bevorzugterweise enthält das Nahrungsergänzungsmittel oder Medikament den Rotbuschextrakt in einer Menge von mindestens 100 mg, weiter bevorzugt von mindestens 200 mg, noch weiter bevorzugt von mindestens 300 mg pro g Nahrungsergänzungsmittel bzw. pro Dosierungseinheit des Medikaments. In einer weiteren bevorzugten Ausführungsform enthält das Nahrungsergänzungsmittel oder Medikament mindestens 1 mg, weiter bevorzugt mindestens 2 mg, noch weiter bevorzugt mindestens 3 mg, noch weiter bevorzugt mindestens 5 mg und am meisten bevorzugt mindestens 10 mg der Verbindung der Formel I pro g Nahrungsergänzungsmittel bzw. pro Dosierungseinheit eines Medikaments.

In einer bevorzugten Ausführungsform der Erfindung wird der Rotbuschextrakts sowie Verbindungen gemäß Formel I sowie deren pharmazeutisch annehmbare Salze, Derivate und Ester mit dem erhöhten Anteil an der Verbindung gemäß Formel I als Arzneimittel zur Vorbeugung oder Behandlung von neurologischen und psychiatrischen Störungen des zentralen Nervensystems, bevorzugt zur Behandlung von Demenzen, Morbus Parkinson, Depressionen und

Schmerzzuständen, insbesondere Alzheimerschen Erkrankung, verwendet.

In einer noch weiter bevorzugten Ausführungsform betrifft die Erfindung die Verwendung von Rotbuschextrakt sowie der Verbindung der Formel I oder deren pharmazeutisch annehmbare Salze und Ester zur Herstellung eines Medikaments zur Vorbeugung und/oder Behandlung von neurologischen und psychiatrischen Störungen des zentralen Nervensystems, worin die neurologischen und psychiatrischen Störungen des zentralen Nervensystems Demenzen, Morbus Parkinson, Depressionen und Schmerzzustände, weiter bevorzugt Alzheimer, sind.

Im Rahmen der vorliegenden Erfindung konnte ein neuer, pharmakologisch wirksamer Naturstoff aus einem Rotbuschextrakt isoliert und anschließend charakterisiert werden. Unerwarteterweise konnte die neue chemische Verbindung nur aus unfermentiertem "grünem" Rotbuschextrakt isoliert werden. Erst die vollständige Charakterisierung der Verbindung ermöglichte den Nachweis geringster Mengen auch in dem fermentierten Rotbuschextrakt.

Das Verfahren zur Isolierung der neuen chemischen Verbindung ist in Beispiel 1 ausführlich beschrieben, die Strukturformel ist in Formel I angegeben. Die Verbindung der Formel I weist sowohl Ähnlichkeiten zur Struktur des Aspalathins als auch des Catechin(4$\alpha$->2)-phloroglucinols (Figur 1) auf. Die genaue Charakterisierung sowie die Strukturaufklärung ist ausführlich in Beispiel 2 beschrieben. Im Vergleich zu den bislang bekannten Flavonoiden weist die neue Verbindung gemäß Formel I ein hohes Molekulargewicht von 740,66 g/mol auf. Derartig hochmolekulare Naturstoffe werden üblicherweise nicht für das Wirkstoffscreening verwendet, da diese aufgrund ihres Molekulargewichts die Blut-HirnSchranke schlecht passieren können. Für den Wirkort Gehirn bzw. zur Behandlung von Krankheiten des zentralen Nervensystems werden derartige Stoffe daher eher als ungeeignet angesehen.

Unerwarteterweise zeigte jedoch eine "bias"-freie Untersuchung im Tele-Stereo-EEG (Elektroenzephalografie) der Ratte eine ausgeprägte, zentralnervöse, pharmakologische Aktivität der Verbindung gemäß Formel I. Die pharmakologischen Untersuchungen zeigten überraschenderweise, dass die Aktivität im Model Tele-Stereo-EEG bei Ratten dosisabhängige Veränderungen der EEG-Frequenzen bewirkt, wie sie nach Gabe klassischer Medikamente zur Behandlung von Demenzen (beispielsweise Galantamin oder Tacrin), Morbus Parkinson (L-DOPA), sowie Schmerzzuständen (beispielsweise Nefopam) bekannt sind.

Die pharmakologische Aktivität der erfindungsgemäßen Verbindung gemäß Formel I wurde im Rahmen der vorliegenden Erfindung mit den bekannten Inhaltsstoffen des Rotbuschextrakts wie Aspalathin, Catechin oder (-)-Epicatechin verglichen. Die experimentellen Untersuchungen sind in Beispiel 3 ausführlich beschrieben und zeigen unerwarteterweise, dass die Wirkung der Verbindung gemäß Formel I nicht mit etwa äquimolaren Mengen an Aspalathin, Catechin oder (-)-Epicatechin erreicht werden kann, obwohl die Verbindung gemäß Formel I strukturelle Ähnlichkeiten zu diesen Naturstoffen aufweist. Die neue erfindungsgemäße Verbindung gemäß Formel I besitzt eine höhere pharmakologische Aktivität als diese bekannten Inhaltsstoffe des Rotbuschextrakts und ist daher besonders geeignet für die Verwendung als Medikament.

Die Isolierung dieser neuen Verbindung mit vorteilhaften pharmakologischen Aktivitäten ermöglicht insbesondere die Herstellung eines. Medikaments oder Nahrungsergänzungsmittels auf Basis der Verbindung gemäß Formel I sowohl als Monopräparat als auch in Kombination mit weiteren Wirkstoffen. Diese weiteren Wirkstoffe können in dieselbe Richtung wirken oder auch völlig andere, das Krankheitsbild auf andere Weise günstig beeinflussende Eigenschaften haben. Hierfür geeignet ist auch die Kombination dieser Verbindung mit anderen in Rotbusch enthaltenen Flavonoiden und Inhaltsstoffen im Gesamtverbund. Insbesondere sind hier Inhaltsstoffe geeignet, die eine antioxidative Wirkung aufweisen.

Eine Diskriminanzanalyse der in vivo-Daten der Verbindung gemäß Formel I zeigte, wie oben erwähnt, eine Verwandtschaft zu den Medikamenten zur Behandlung von Demenzen, Morbus Parkinson, Depression und Schmerzzuständen. Da diese Medikamente ein breites Nebenwirkungsspektrum aufweisen, ist die Verwendung der Verbindung gemäß Formel I als Medikament vorteilhaft, da Naturstoffe üblicherweise eine geringere Nebenwirkungsrate erwarten lassen. Unerwarteterweise überschreitet die neue Substanz oder deren Metabolite offensichtlich zudem die Blut-Hirnschranke. Dies ist nicht allgemein üblich für Flavonoide.

Desweiteren wird ein Herstellungsverfahren für Rotbuschextrakte sowie für die neu identifizierte pharmakologisch wirksame Verbindung zur Verfügung gestellt. Durch das erfindungsgemäße Verfahren ist es möglich, einen besonders geeigneten Pflanzenextrakt zur Behandlung der vorstehend genannten Krankheiten zur Verfügung zu stellen.

Der erfindungsgemäß hergestellte Rotbuschextrakt weist einen Gehalt an der Verbindung gemäß Formel I von mindestens 1 Gew.-%, weiter bevorzugt mindestens 1,5 Gew.-%, noch weiter bevorzugt mindestens 2 Gew.-%, weiter bevorzugt mindestens 2,5 Gew.-%, noch weiter bevorzugt mindestens 3 Gew.-% und am meisten bevorzugt mindestens 5 Gew.-%, auf.

In einer ganz besonders bevorzugten Ausführungsform wird ein hochkonzentrierter Rotbuschextrakt eingesetzt. Ein hochkonzentrierter Rotbuschextrakt beinhaltet wenigstens 10 Gew.-% und bevorzugt wenigstens 20 Gew.-% der Verbindung der Formel I. In einer besonderen Ausführungsform beinhaltet der hochkonzentrierte Rotbuschextrakt wenigstens 50 Gew.-% der Verbindung der Formel I.

Ein erfindungsgemäßes Verfahren zur Herstellung der Verbindung gemäß Formel I weist folgende Schritte auf (siehe auch Beispiel 1):

- Bereitstellen getrockneter und zerkleinerter, unfermentierter Rotbusch-Rohware,
- Extrahieren der bereitgestellten Rohware mit einem Extraktionsmittel bestehend aus einer Mischung aus einem Alkohol, bevorzugterweise Methanol, und/oder Wasser für eine vorbestimmte Extraktionsdauer bei einer Temperatur von bis zu 90°C, vorzugsweise bis zu 60°C,
- Filtrieren des Extrakts,
- Einengen des filtrierten Extrakts unter reduziertem Druck,
- Reinigen des Extrakts in drei Schritten:

  - grobe Reinigung durch Chromatographie an einer Sephadex LH20-Säule
  - Feinreinigung durch Chromatographie an einer weiteren Sephadex LH20-Säule
  - Auftrennung an einer lipophilen c18-HPLC-Säule.

In einer besonders bevorzugten Ausführungsform wird bei dem Extraktionsschritt des erfindungsgemäßen Verfahren Ascorbinsäure in einer Menge von 0,001 bis 1 Gew.-%, bevorzugt 0,01 bis 0,2 Gew.-% zugegeben. Die Gewichtsprozent beziehen sich auf das Gewicht der zu extrahierenden Droge, also Pflanzenteile wie getrocknete Blätter oder Stengel.

Wenn Präparate mit erhöhtem Gehalt an Verbindungen gemäß Formel I gewünscht sind, kann der Extrakt auch nur mit einer Chromatographiesäule aufgetrennt werden.

Bevorzugterweise ist der Feuchtegehalt der bereitgestellten Rotbusch-Rohware 4% oder kleiner, da auf diese Weise eine Autofermentation des Ausgangsmaterials verhindert wird.

Für die Erzielung einer hohen Ausbeute an der Verbindung der Formel I wird als Extraktionsmittel gemäß einer bevorzugten Ausgestaltung des erfindungsgemäßen Verfahrens eine Alkohol/Wasser-Mischung im Verhältnis 50:50 bis 80:20 verwendet, je nach verwendetem Alkohol (Methanol, Ethanol, Propanol, Propan-2-ol sind geeignet). Bevorzugterweise wird eine Methanol/Wasser-Mischung von 50:50 verwendet. Bei dieser bevorzugten Ausführungsform des erfindungsgemäßen Verfahrens beträgt das Verhältnis von Rohware zu Extraktionsmittel vorzugsweise ca. 1:6, und der Schritt des Extrahierens erfolgt vorzugsweise bei erhöhter Temperatur (über 40°C) 1 Stunde lang, ist aber auch bei Raumtemperatur und einer Dauer von beispielsweise 2 bis 5 Stunden möglich.

Das Einengen des filtrierten Extrakts erfolgt vorzugsweise bei einem Druck von weniger als 300 mbar. Die Temperatur beträgt bei dem Einengungsschritt vorzugsweise maximal 40°C.

Die Herstellung des erfindungsgemäßen Rotbuschextrakts erfolgt nach dem vorstehend beschriebenen Verfahren, wobei der Rotbuschextrakt nach dem Einengen bis zur Trockne (ohne nachfolgende chromatographische Reinigung) erhalten wird.

Das Messverfahren zur Bestimmung des Gehalts des Rotbuschextrakts an der Verbindung gemäß Formel I wird in Beispiel 4 ausführlich beschrieben.

Die Verbindung gemäß Formel I, deren pharmazeutisch annehmbare Salze, Derivate und Ester, sowie der erfindungsgemäße Rotbuschextrakt sind insbesondere zur Behandlung von Krankheiten des zentralen Nervensystems, bevorzugt Demenzen, Morbus Parkinson, Depressionen, Schmerzzuständen und als Zellschutz-Antioxidans bzw. "Radikalfänger" geeignet. Die Behandlung von Demenzen wie Altersdemenz oder Morbus Alzheimer ist besonders bevorzugt.

Erfindungsgemäß können die neuen Verbindungen als Einzelwirkstoff oder in Kombination mit weiteren Wirkstoffen, in der Form gemäß Formel I oder als pharmazeutisch annehmbare Salze oder Komplexe sowie Ester oder Derivate eingesetzt werden. Geeignete Derivate, Salze, Komplexe und Ester sowie deren Herstellung sind dem Fachmann bekannt. Die Herstellung pharmazeutisch annehmbarer Salze (Hydrochlorid, Succinate, Citrat, Tartrat etc.) ist dem Fachmann ebenfalls bekannt. Als Salzbildner kommen alle üblichen pharmazeutisch annehmbare Säuren bzw. Anionen in Frage. Desweiteren sind Kopplungen an Säuren wie beispielsweise Ferulasäure, Chinasäure, Kaffeesäure, Gluconsäure, Chlorogensäure und verwandte Verbindungen möglich. Insbesondere ist eine Kopplung an Gluconsäure bevorzugt. Die Kopplungsprodukte der Verbindung gemäß Formel I an die vorstehenden Säuren werden als pharmazeutisch verträgliche Derivate bezeichnet. Bevorzugterweise wird die Verbindung aber als Molekül gemäß Formel I verwendet.

In einer anderen, ebenfalls bevorzugten Ausführungsform werden die Extrakte dadurch hergestellt, dass ein etwas hydrophileres Lösungsmittelgemisch verwendet wird. Dieses Lösungsmittelgemisch umfasst wenigstens einen Alkohol, bevorzugt ausgewählt aus Methanol, Ethanol, n-Propanol oder 2-Propanol und Wasser. Der Alkoholgehalt liegt zwischen 10 und 50 Gew.-% Alkohol. Wenn dieses Lösungsmittelgemisch gewählt wird, wird der Gesamtflavonoidanteil erhöht im Vergleich zu einem Lösungsmittelgemisch, bei dem höhere Anteile an Alkohol eingesetzt werden. Bei dieser Ausführungsform liegt der Alkoholgehalt zwischen 10 und 60 % (Vol/Vol), bevorzugt zwischen 10 und 50 % (Vol/Vol). Noch bevorzugter beträgt der Alkoholgehalt zwischen 15 und 40 % und ganz besonders bevorzugt zwischen 20 und 30 % (Vol/Vol).

Bei dieser Ausführungsform des Verfahrens wird als Ausgangsdroge "grüner" Rotbusch mit einem möglichst

geringen Restfeuchtegehalt, der bevorzugt weniger als 5 % (Wasser pro Gesamtgewicht) beträgt, eingesetzt. Das Ausgangsmaterial, nämlich Blätter und Zweigspitzen von Aspalathus linearis (nicht fermentiert) wird besonders schnell und schonend getrocknet und anschließend mit einer Alkohol-Wasser-Mischung extrahiert, wobei der Wassergehalt (>60 % Vol/Vol) beträgt. In besonders bevorzugten Ausführungsformen beträgt der Alkoholgehalt 15 bis 25 %, ganz besonders bevorzugt 20 % Methanol oder 25 bis 35 %, besonders bevorzugt 30 % Ethanol.

In einer ganz besonders bevorzugten Ausführungsform wird die getrocknete Ausgangsdroge zunächst mit reinem Alkohol versetzt und nach einer Einweichphase, die üblicherweise zwischen 30 und 60 Minuten dauert, die entsprechende Wassermenge zugegeben. Nach der Extraktion wird der Extrakt filtriert und das Filtrat unter reduziertem Druck zur Trockne eingeengt. Hieran schließt sich bevorzugt ein chromatographischer Reinigungsschritt an, wobei die Aufreinigung bevorzugt durch Anwendung einer Sephadex-Säulenchromatographie erfolgt.

Bei dieser Ausführungsform werden Verbindungen der Formel I nahezu vollständig extrahiert, wobei gleichzeitig ein hoher Anteil der Gesamtflavonoide extrahiert wird. Die Extrakte (vor weitergehender Reinigung mit Chromatographie-Verfahren) weisen einen Gehalt an Verbindungen der Formel I auf, der zwischen 2,5 und 5 % (Gewicht bezogen auf Trockenextrakt) beträgt. Der Gesamtflavonoidgehalt, als die Summe der Aspalathin-Artigen, der Rutosid-Artigen und Vitexin-Artigen ohne Verbindung gemäß Formel I liegt zwischen 15 und 30 Gew.-% bezogen auf den Trockenextrakt. Das Verhältnis Vitexin-artige (= C-Gycoside) zu Rutosid-Artigen ($\leq$ 1,6). Der Gehalt an Aspalathin-Artigen beträgt 14 bis 25 %, bezogen auf das Gewicht des Trockenextraktes.

Bei dieser Art der Ausführung des Extraktionsverfahrens wird der Gesamtflavonoidgehalt des Extraktes erhöht. Üblicherweise weist ein derartiger Extrakt (ohne zusätzliche Reinigung durch Säulenchromatographie) wenigstens etwa 20 Gew.-% Gesamtflavonoide auf. Die folgenden Hauptkomponenten konnten in dem Extrakt identifiziert werden:

a) Verbindung der Formel I;
b) Substanzgruppe der Chalcone (Aspalatin und Nothofagin);
c) Rutosid-Gruppe. Hierunter fallen Flavonoide, die Quercetin als Bestandteil enthalten, Flavonoide, die eine Zucker über eine C-O-C-Verknüpfung enthalten (Quercetin ist das Aglycon von Rutin) sowie
d) die Vitexin-Gruppe. Hierunter werden Flavonoide verstanden, die Zucker über eine C-C-Verbindung enthalten. Vitexin ist das Aglycon von Apigenin, z.B. Vitexin (Apigenin-8-C-glucosid), Isovitexin (Apigenin-6-C-glucosid), Orientin (Luteolin-8-C-glucosid), Isoorientin (Lutoelin-6-C-glucosid).

Charakteristisch für diesen Rotbuschextrakt ist unter anderem das Gewichtsverhältnis der einzelnen Gruppen zueinander. So liegt das Verhältnis der Gruppe der Vitexine zu der Rutosidgruppe bezogen auf die Gewichtsanteile in einem Verhältnis von 1:2 bis 1:50, bevorzugt in einem Verhältnis zwischen 1:3 und 1:10.

Der erfindungsgemäße Extrakt unterscheidet sich von solchen Extrakten für die innerliche Anwendung, die bei Teegetränken, also bei rein wässriger Extraktion erhalten werden durch höhere Gehalte an den erwähnten Verbindungen. Von Extrakten, die für die äußerliche Anwendung, beispielsweise in der Kosmetik vorgesehen sind, unterscheiden sich die erfindungsgemäßen Extrakte durch ein anderes Verhältnis der Flavonoidgruppen zueinander. Extrakte, die für die äußerliche Anwendung bestimmt sind, werden durch eine Extraktion mit höherem Ethanolgehalt (wenigstens etwa 80 % Ethanol) erhalten. Der Extrakt, erhalten mit 80 % Ethanol, wurde auf einen möglichst hohen Aspalathingehalt optimiert. Das Verhältnis der drei Flavonoidgruppen (Aspalathin-artige, Rutosid-artige und Vitexin-artige = C-Glycoside) wird durch die relativ lipophile Extraktion gegenüber dem Ausgangszustand in der Droge verändert. Erkennbar wird dies besonders deutlich am Verhältnis der Vitexin-artigen zu den Rutosid-artigen Flavonoiden.

Der erfindungsgemäße Rotbuschextrakt sowie die erfindungsgemäße Verbindung gemäß Formel I bzw. deren Salze, Derivate und Ester, bevorzugt die natürlich vorkommende Verbindung gemäß Formel I, können auf an sich bekannte Weise zu Arzneimitteln und/oder zu Nahrungsergänzungsmitteln mit gesundheitsfördernden Eigenschaften verarbeitet werden. Der erfindungsgemäße Rotbuschextrakt sowie die erfindungsgemäße Verbindung gemäß Formel I bzw. deren Salze, Derivate und Ester können z.B. in Form von Tabletten, Kapseln, Pillen, Dragees, Granulaten, Pulvern, Lutschpastillen und flüssigen Darreichungsformen wie z.B. Getränken formuliert werden. Bevorzugt ist auch die Verwendung in Nahrungsergänzungsmitteln, insbesondere für Heiß- und Kaltgetränke, oder als löslicher Tee.

Bevorzugterweise werden die Nahrungsergänzungsmittel bzw. Arzneimittel oral verabreicht, wobei auch beispielsweise die topische, parenterale, intravenöse, intramuskuläre, subkutane, nasale, inhalative, rektale oder transdermale Applikation möglich ist.

Der erfindungsgemäße Rotbuschextrakt sowie die erfindungsgemäßen Arzneimittel bzw. Nahrungsergänzungsmittel können zusätzlich bevorzugt weitere Wirkstoffe, die die Wirkung des Rotbuschextrakts oder der erfindungsgemäßen Verbindung gemäß Formel I bzw. deren Salze verstärken oder die bei den benannten Krankheiten auftretenden Symptome oder Zustände ergänzend positiv beeinflussen (z.B.: weitere Radikalfänger, verschiedene enzymhemmende Stoffe, Vitamine, Lecithine, omega-3-Fettsäuren und Stoffe, die die Funktionen des Gehirns positiv beeinflussen), enthalten.

Desweiteren können pharmazeutisch annehmbare Hilfs- und Trägerstoffe verwendet werden. Geeignete Hilfsstoffe sind dem Fachmann bekannt und umfassen beispielsweise Füllstoffe, Sprengmittel, Gleitmittel, Bindemittel, Benetzungsmittel, etc.

Geeignete Gleitmittel sind z.B. Silikat, Talk, Stearinsäure, Magnesium- oder Calciumstearat und/oder Polyethylenglykole.

Als Bindemittel können beispielsweise Stärken, Gummi arabicum, Gelatine, Methylcellulose, Carboxymethylcellulose oder Polyvinylpyrrolidon verwendet werden.

Aufschlussmittel sind beispielsweise Stärke, Alginsäure, Alginate oder Natriumstärkeglykolate, aufschäumende Mischungen.

Als Benetzungsmittel können beispielsweise Lecithin, Polysorbate oder Laurylsulfate verwendet werden.

Desweiteren können auch Farbstoffe und Süßungsmittel in den Formulierungen enthalten sein.

Die pharmazeutischen Zubereitungen können in bekannter Weise hergestellt werden, z.B. mittels Mischen, Granulieren, Tablettieren, Zuckerbeschichtungs- oder Überzugsbeschichtungsverfahren.

Die flüssigen Dispersionen und/oder Lösungen zur oralen Verabreichung können z.B. Getränke, Tropfen, Sirupe, Emulsionen und Suspensionen sein.

Der Sirup kann als Träger z.B. Saccharose oder Saccharose mit Glycerin und/oder Mannitol und/oder Sorbitol enthalten.

Die Suspensionen und die Emulsionen können als Träger z.B. ein natürliches Harz, Agar, Natriumalginat, Pektin, Methylcellulose, Carboxymethylcellulose oder Polyvinylalkohol enthalten.

Die Suspensionen oder Lösungen für intramuskuläre Injektionen können zusammen mit dem Wirkstoff einen pharmazeutisch annehmbare Träger, z.B. steriles Wasser, Olivenöl, Ethyloleat, Glykole, z.B. Propylenglykol, und, falls gewünscht, eine geeignete Menge an Lidocain-Hydrochlorid enthalten.

Die Lösungen zur intravenösen Injektion oder Infusion können als Träger z.B. steriles Wasser enthalten oder sie können bevorzugt in Form von sterilen, wässrigen, isotonischen Salzlösungen vorliegen.

Die Suppositorien können zusammen mit dem Wirkstoff einen pharmazeutisch annehmbare Träger, z.B. Kakaobutter, Polyethylenglykol, einen Polyoxyethylensorbitol-Fettsäureester oder Lecithin enthalten.

Zusammensetzungen für die topische Applikation, z.B. Cremes, Lotionen oder Pasten, können durch Mischen des Wirkstoffs mit einem herkömmlichen ölhaltigen oder emulgierenden Träger hergestellt werden.

Der erfindungsgemäße Rotbuschextrakt sowie die erfindungsgemäße Verbindung gemäß Formel I bzw. deren Salze, Derivate und Ester können in üblichen Mengen in den Nahrungsergänzungs- bzw. Arzneimitteln verwendet werden. In Lösung werden bevorzugterweise 0,001 bis 10 Gew.-% der erfindungsgemäßen Verbindung gemäß Formel I bzw. deren Salze, weiter bevorzugt 0,1 bis 7 Gew.-% und besonders bevorzugt 1 bis 5 Gew.-%, eingesetzt. In einer besonderen Ausführungsform werden in Lösung 0,02 bis 1 Gew.-% der erfindungsgemäßen Verbindung gemäß Formel I bzw. deren Salze eingesetzt.

Der erfindungsgemäße Rotbuschextrakt wird bevorzugt in Mengen eingesetzt, die einer Menge an der Verbindung gemäß Formel I von 1 bis 1000 mg, weiter bevorzugt 10 bis 600 mg, noch weiter bevorzugt 50 bis 400 mg und am meisten bevorzugt 50 bis 250 mg entsprechen.

Wenn ein Rotbuschextrakt eingesetzt wird, der einen sehr hohen Anteil an der Verbindung gemäß Formel I aufweist, kann ein derartiger Extrakt bei Arzneimitteln bei Nahrungsergänzungsmitteln in einer Menge eingesetzt werden, die zwischen 3 und 600 mg, bevorzugt zwischen 5 und 100 mg und besonders bevorzugt zwischen 10 und 50 mg pro Tagesdosis liegt.

Die oben beschriebenen Nahrungsergänzungsmittel bzw. Arzneimittel können mit herkömmlichen Methoden hergestellt und in einer pharmazeutisch geeigneten Form verabreicht werden.

Die erfindungsgemäß bevorzugten, festen Nahrungsergänzungs- bzw. Arzneimittel können zusätzlich 1 bis 95 Gew.-%, bevorzugt 1 bis 50 Gew.-%, stärker bevorzugt 1 bis 20 Gew.-%, besonders bevorzugt 1 bis 10 Gew.-% Füllstoffe enthalten.

Als Füllstoffe können eine oder mehrere Verbindungen verwendet werden, die einen Teil des Materials zum Erreichen der erforderlichen und gewünschten Tabletten- oder Kapselmasse liefern. Einsetzbar sind unter anderem mikrokristalline Cellulose in verschiedenen Partikelgrößen, insbesondere mit einer mittleren Partikelgröße im Bereich von 20 $\mu$m bis 200 $\mu$m, insbesondere im Bereich von 50 $\mu$m bis 150 $\mu$m, wie z.B. etwa 100 $\mu$m, wie die bekannten Avicel-Produkte wie Avicel PH-101 und PH-102. Weitere geeignete Füllstoffe sind z.B. Maisstärke, Kartoffelstärke, Laktose, Cellaktose (eine Mischung aus Cellulose und Laktose), Calciumphosphat, Dextrose, Mannit, Maltodextrin, Isomalt, Siliziumdioxid (Aerosil), gegebenenfalls auch Sorbit und Saccharose. Falls eine Direktverpressung vorgesehen ist, sollte bei der Auswahl der Füllstoffe darauf geachtet werden, dass Qualitäten verwendet werden, die für die Direktverpressung von Tabletten geeignet sind. Dies wird bei den kommerziellen Produkten jeweils vom Hersteller angegeben bzw. kann durch einfache Versuche überprüft werden. Das am stärksten bevorzugte Füllmittel ist mikrokristalline Cellulose (Handelsprodukte sind beispielsweise Avicel, Vivapur und Emcocel).

Geeignete Sprengmittel sind im Stand der Technik bekannt. Sprengmittel werden häufig auch mit dem englischen Begriff "Disintegrants" bezeichnet. Erfindungsgemäß bevorzugte Sprengmittel sind z.B. Crospovidone (Kollidon

CL) und Stärke bzw. vorverkleisterte Stärke, insbesondere das Handelsprodukt "Starch 1500". Weitere geeignete Stärken sind z.B. unter den Bezeichnungen Lycatab PGS, Prejel und Sepistab ST 200 kommerziell erhältlich.

Weiterhin können auch die bekannten sogenannten "Super Disintegrants" verwendet werden, wie Croscarmellose Natrium (z.B. Ac-Di-Sol, u.a.) und Carboxymethylstärke Natrium (z.B. Explotab, Primojel, u.a.). Besonders bevorzugt sind Stärken wie Starch 1500.

Der Gehalt an Sprengmittel ist in der Regel 1 bis 25 Gew.-%, vorzugsweise 1 bis 20 Gew.-%, insbesondere 2 bis 15 Gew.-%. Geeignete Bereiche für den Gehalt an Sprengmittel sind auch z.B. 2 bis 5 Gew.-% oder 15 bis 20 Gew.-%, in Abhängigkeit der verwendeten Sprengmittel, Füllstoffe und übrigen Zusatzstoffe.

Erfindungsgemäß kann die Zusammensetzung als Gleitmittel eine oder mehrere Verbindungen enthalten, die die Herstellung und die Verarbeitung der Tablette unterstützen. Verwendbare Gleitmittel sind unter anderem Stearinsäure und deren Derivate, wie Calciumstearat, und insbesondere Natriumstearylfumarat (das z.B. unter der Bezeichnung Pruv kommerziell erhältlich ist) und Magnesiumstearat, Glycerolmono-, -di-und insbesondere -tristearat, hydriertes Pflanzenöl (z.B. Lubritab, Dynasan, Sterotext) oder ein Polyethylenglykol (z.B. Lutrol, Carbowax).

Der Gehalt an Gleitmittel ist in der Regel 0,1 bis 4 Gew.-%, vorzugsweise 0,2 bis 4 Gew.-%.

Optional kann die erfindungsgemäße pharmazeutische Zusammensetzung ein oder mehrere Fließregulierungsmittel umfassen. Geeignete Fließregulierungsmittel sind Magnesiumtrisilikat, Talk und insbesondere Siliziumdioxid (z.B. Aerosil). Falls die Zusammensetzung ein Fließregulierungsmittel umfasst, ist dieses in der Regel in einer Menge von 0,5 bis 5 Gew.-%, vorzugsweise 1 bis 4 Gew.-%, insbesondere 2 bis 3 Gew.-% vorhanden.

Die erfindungsgemäßen pharmazeutischen Zusammensetzungen können ebenfalls noch Stabilisatoren für den Wirkstoff enthalten, wie Ascorbinsäure, Zitronensäure, Weinsäure, Milchsäure, etc., bevorzugt Ascorbinsäure oder Zitronensäure. Der Gehalt an Stabilisator (falls vorhanden) ist in der Regel im Bereich von 0,1 bis 10 Gew.-%, 0,5 bis 10 Gew.-%, bevorzugt 1 bis 3 Gew.-%.

Die erfindungsgemäßen pharmazeutischen Zusammensetzungen können weitere übliche pharmazeutisch verträgliche Zusatz- und Hilfsstoffe enthalten, bevorzugt enthalten sie aber außer den vorstehend angegebenen (Füllstoff, Sprengmittel, Schmiermittel und gegebenenfalls Fließregulierungsmittel und Stabilisator) keine weiteren Hilfsstoffe.

Einige Füllstoffe wie mikrokristalline Cellulose können auch als Bindemittel dienen. Auch Füllstoffe mit Bindemittelfunktion zählen daher im Rahmen dieser Anmeldung zu den Füllstoffen.

Liegt die erfindungsgemäße pharmazeutische Zusammensetzung als Tablette vor, kann sie mit einem oder mehreren Beschichtungsmitteln filmbeschichtet sein. Verwendbare Beschichtungsmittel sind Schellack und Schellack-Mischungen, Hypromellose (Hydroxypropylmethylcellulose), Polyvinylalkohol, Natriumcarboxymethylcellulose und verschiedene Methacrylsäurepolymere (Eudragite), wobei Hypromellose, Eudragite, Schellack und Schellack-Mischungen bevorzugt sind. Das Beschichten der Tabletten erfolgt auf übliche Art und Weise. In der Beschichtung können außer dem Beschichtungsmittel weitere übliche Bestandteile von Tablettenbeschichtungen wie Weichmacher, Pigmente, Porenbildner oder Suspensionsstabilisatoren vorhanden sein wie z.B. Polyethylenglykol (PEG), Talk oder Titandioxid und gegebenenfalls auch Laktose.

Das Tablettengewicht ist nicht besonders eingeschränkt, üblich sind Tabletten mit 100 mg bis 500 mg bei Verwendung von reinen Wirkstoffen und 250 mg bis 1500 mg, 500 mg bis 1500 mg bei Verwendung von Extrakten und Pflanzenpulvern. In Kapseln werden Mengen von 100 mg bis 1000 mg verwendet.

Die Dosierungseinheit des Arzneimittels oder Nahrungsergänzungsmittels kann beispielsweise enthalten:

- bei peroralen Arzneiformen:

  bevorzugt 1 bis 1000 mg, bevorzugter 40 bis 800 mg, besonders bevorzugt 150 bis 500 mg, noch bevorzugter 300 bis 600 mg, Rotbuschextrakt pro Tagesdosis. Bei Verwendung der Verbindung gemäß Formel I, sowie deren pharmazeutisch annehmbaren Salze, Derivate und Ester werden 1/100 bis 1/20 der vorstehenden Mengen eingesetzt.

Die Tagesdosis kann beispielsweise in 1 bis 3 Einzeldosen, vorzugsweise in zwei Einzeldosen, täglich verabreicht werden. Es kann auch vorgesehen sein, dass 1-10 Einzeldosen Rotbuschextrakt enthaltend die Verbindung gemäß Formel 1 täglich verabreicht werden.

- bei parenteralen Arzneiformen (zum Beispiel intravenös, subkutan, intramuskulär):

  bevorzugt 3 bis 60 mg, besonders bevorzugt 10 bis 30 mg, Wirkstoff pro Tagesdosis.

Die Tagesdosis kann beispielsweise in 1 bis 3 Einzeldosen, vorzugsweise in einer Einzeldosis, täglich verabreicht werden.

- bei Arzneiformen zur rektalen Applikation:

  bevorzugt 40 bis 80 mg, besonders bevorzugt 60 mg, Wirkstoff gemäß Formel I der Extrakt pro Tagesdosis.

Die Tagesdosis kann beispielsweise in 1 bis 3 Einzeldosen, vorzugsweise in einer Einzeldosis, täglich verabreicht werden.

- bei Arzneiformen zur Applikation auf die Haut und Schleimhäute (z.B. Lösungen, Lotionen, Emulsionen, Salben usw.):

  bevorzugt 40 bis 80 mg Wirkstoff, besonders bevorzugt 60 mg, Wirkstoff pro Einzeldosis. Bezieht man den Gehalt an Verbindung gemäß Formel I auf die fertige Lösung, Lotion, Emulsion oder Salbe, dann beträgt der Gewichtsanteil bezogen auf derartige salbenartige Arzneimittel zwischen 0,05 und 20 Gew.-%, bevorzugt zwischen 0,2 und 1 Gew.-% an Verbindung der Formel I bezogen auf die cremeartige Präparation.

Die Tagesdosis kann beispielsweise in 1 bis 6 Einzeldosen, vorzugsweise in 1 bis 3 Einzeldosen, täglich verabreicht werden.
Bei Verwendung eines pharmazeutisch annehmbare Salzes muss die verwendete Menge in dem Fachmann bekannter Weise entsprechend angepasst werden.

**Beschreibung der Figuren**

**Figur 1** zeigt die Strukturformel von Aspalathin (1) und Catechin(4a->2)-phloroglucinol (2).

**Figur 2** zeigt die Nummerierung der Atome in der Verbindung gemäß Formel I.

**Figur 3** zeigt die Zuordnung der NMR(nuclear magnetic resonance)-Signale zu den Atomen mit den Nummern gemäß Figur 2. Erklärung der Legende: *)/**)/+)/++)/§)/§§)/#)##) = Werte können untereinander ausgetauscht werden, A). B) = Summe des Integrals bzw. chemische Verschiebung der Gruppe nur einmal angegeben.

**Figur 4** zeigt mittels NMR-Experimenten charakterisierte Strukturfragmente A-D der Verbindung gemäß Formel I.

**Figur 5** zeigt Tabelle 2 mit $^{13}C$-NMR-Daten der Verbindung gemäß Formel I im Vergleich zu Aspalathin und Catechin(4$\alpha$->2)-phloroglucinol. Aufgrund fehlender $^{13}C$-NMR-Literaturdaten zu Aspalathin erfolgte die Zuordnung der Signale für das bei HWI ANALYTIK GMBH isolierte Aspalathin in Anlehnung an Daten aus: Ho et al., Phytochemistry 1980, 19, 476-477. Die Daten von Catechin(4$\alpha$->2)-phloroglucinol wurden Matthewis et al., J. Agric. Food. Chem. 1997, 45, 1195-1201 entnommen. Erklärung der Legende: *)/**)/+)/++)/§)/§§)/#)##) = Werte können untereinander ausgetauscht werden.

**Figur 6** zeigt den Nachweis der stabilen Versuchsbedingungen bei Verabreichung von Salzlösung an Fischer-344 Ratten nach Verabreichung der Verbindung gemäß Formel I. Auf der y-Achse ist die Zeit in Stunden nach Applikation aufgetragen. Die x-Achse gibt die Frequenzbereiche nach der Fast Fourier Transformation der Daten an: delta, theta, alpha1, alpha2, beta1 und beta2.

**Figur 7** zeigt die Wirkung von 3 mg/kg der Verbindung gemäß Formel I auf EEG Frequenzen bis 5 Stunden nach Applikation. Mittelwerte von n=6 Tieren. Sterne kennzeichnen die statistische Signifikanz, berechnet nach Ahrens und Läuter, 1974. *=p<0.01; **=p<0.05; ***=p<0.025.

**Figur 8** zeigt die Wirkung von 6 mg/kg der Verbindung gemäß Formel I auf EEG Frequenzen bis 5 Stunden nach Applikation. Mittelwerte von n=6 Tieren. Sterne kennzeichnen die statistische Signifikanz, berechnet nach Ahrens und Läuter, 1974. *=p<0.01; **=p<0.05; ***=p<0.025.

**Figur 9** Wirkung der Verbindung der Formel I im Vergleich mit seinen Molekülbestandteilen Aspalathin, Catechin bzw. Epicatechin. Bei etwa äquimolarer Gabe kann die Wirkung auf Grund der einzigartigen Struktur nur von der Verbindung gemäß Formel I, nicht jedoch durch Teile des Moleküls erreicht werden.

**Figur 10** zeigt die Wirkung von 6 mg/kg der Verbindung der Formel I auf EEG Frequenzen während der ersten Stunde nach Applikation. Mittelwerte von n=6 Tieren. Man beachte die Ähnlichkeit zu Medikamenten die zur Behandlung von Demenz, Morbus Parkinson, Depression und Schmerzzuständen eingesetzt werden: L-DOPA (Morbus Parkinson); Tacrin und Galantamin (Morbus Alzheimer; Nefopam (Schmerzbehandlung).

**Figur 11** zeigt das Ergebnis der Motilitätsmessung, die gleichzeitig mit der Messung der Feldpotentiale vorgenommen wurde. Angaben erfolgen in % des Ausgangswertes vor Substanzgabe. Nur (-) Epicatechin verursacht eine deutliche Erhöhung der Motilität.

**Figur 12** zeigt ein UV-Spektrum der Verbindung mit der Formel (G110907SA).

**Figur 13** zeigt ein UV-Spektrum von Aspalatin.

**Figur 14** zeigt ein UV-Spektrum von Rutosid.

**Figur 15** zeigt ein UV-Spektrum von Orientin.

**Figur 16** stellt ein UV-Spektrum von Homoorientin dar.

**Figur 17** stellt ein UV-Spektrum von Vitexin dar.

Die vorliegende Erfindung wird durch die nachfolgenden Beispiele näher erläutert.

**Beispiel 1 -** Isolierung der Verbindung gemäß Formel I

Zur Herstellung der Extrakte aus der Droge wird unfermentierter grüner Rotbusch vom Hersteller Rooibos Ltd. Clanwilliam Süd-Afrika verwendet.

Als Ausgangsmaterial werden schonend auf einen Feuchtegehalt von kleiner als 10% (besser kleiner als 4%) getrocknete, unfermentierte und zerkleinerte Blätter und/oder Zweigspitzen von Aspalathus linearis verwendet.

Dieses Rohmaterial wird mit einer Mischung aus Methanol und Wasser im Verhältnis 50:50 (Volumenteile) bei 60°C für 1 Stunde lang unter Rotation extrahiert, wobei das Verhältnis Rohware:Lösemittel 1:7 beträgt. Danach wird die Flüssigkeit von den Pflanzenteilen abfiltriert und die Pflanzenteile erneut auf die gleiche Weise extrahiert und filtriert.

Die beiden Filtrate werden vereinigt und bei vermindertem Druck (220 mbar) und 55°C vom Methanol befreit. Die verbleibende wässrige Lösung wird mit Wasser zum 5-fachen des Gewichts der eingesetzten Menge getrockneter Pflanzenteile verdünnt und einer Flüssigflüssig-Verteilung unterzogen.

3 Liter der wässrigen Lösung werden 4 mal mit jeweils 1,5 L wassergesättigtem n-Butanol ausgeschüttelt und die vereinigten Butanolphasen unter reduziertem Druck zur Trockne gebracht. Die Ausbeute beträgt ca. 10% der eingesetzten Menge getrockneter Pflanzenteile.

Anschließend erfolgt zunächst eine Grob- und danach eine Feintrennung des Butanolextrakts.

Grobtrennung:

Ca. 50 g Butanolextrakt werden über eine Sephadex LH20-Säule (6 cm Innendurchmesser und 80 cm Füllhöhe = 2260 ml Sephadex LH20) mit 50 Vol% Methanol chromatographiert. Dazu werden die 50 g Butanolextrakt in 400 ml mobiler Phase gelöst und auf die Trennsäule gegeben. Die Säule wird mit einem Fluss von 1,8 ml/Min. so lange gewaschen, bis 3 L Eluat abgetropft sind. Die Säulenfüllung wird nach dem vollständigen Abtropfen der restlichen mobilen Phase entnommen und in 3 L 100%-igem Methanol 10 Minuten lang ausgerührt (extrahiert). Die stationäre Phase wird abfiltriert und das Eluat getrocknet. Der Rückstand beträgt ca. 0,5 bis 1 % der eingesetzten Menge Pflanzenteile = Methanolextrakt.

Erste Feintrennung

Ca. 4 g Methanolextrakt werden über eine Sephadex LH20-Säule (3,5 cm Innendurchmesser und 50 cm Füllhöhe = 480 ml Sephadex LH20) mit 80 Vol% Methanol chromatographiert. Dazu werden die 4 g Butanolextrakt in 40 ml mobiler Phase gelöst und auf die Trennsäule gegeben. Die Säule wird mit einem Fluss von 2,4 ml/Min. betrieben und Fraktionen von je 10 Min. Dauer = 24 ml Eluat aufgefangen. Die gesuchte Substanz befindet sich in den Fraktionen Nr. 48-65. Die Ausbeute beträgt bei 4 g Methanolextrakt ca. 0,5 bis 1 g.

Zweite Feintrennung:

Trennsäule: 250 x 30 mm
Stationäre Phase: Reprosil C18 Aqua 10 µm
Mobile Phase: Methanol 35% (V/V)
Fluss: 1,5 ml/Min.
Fraktionsgröße: 10 Min. = 15 ml
Substanz I befindet sich in den Fraktionen 41 bis 52.
Die vereinigten Fraktionen 41 bis 52 werden lyophilisiert.
Ausbeute ca. 125 mg Substanz I aus 4 g Methanolextrakt.
Chromatographische Reinheit ca. 97% (HPLC).

**Beispiel 2 -** Strukturaufklärung der Verbindung gemäß Formel I

Die durch säulenchromatographische Auftrennung erhaltene Verbindung wurde mit den nachfolgenden Geräten charakterisiert und die nachfolgend aufgezählten Messwerte erzielt.

Figur 2 zeigt die Nummerierung der Atome in der erfindungsgemäßen Verbindung gemäß Formel I. In Figur 3 ist Tabelle 1 mit der Zuordnung der NMR-Daten dargestellt.

Die NMR-Daten wurden wie nachfolgend beschrieben durchgeführt und interpretiert.

<u>$^1$H-NMR (d6-DMSO (Dimethylsulfoxid))</u>

Das $^1$H-NMR der Substanz in DMSO lieferte ein Spektrum, in dem durch Zugabe von deuteriertem Wasser neun austauschende phenolische Protonen sowie fünf aliphatische HO-Protonen durch Signalverkleinerung (H/D-Tausch) identifiziert werden konnten. Zusätzlich treten acht Protonen im olefinischen Bereich von 5,5 bis 8,0 ppm sowie neun Protonen im für $-CH_xOH/-CH_xOR$-Gruppen (x = 1, 2) typischen Verschiebungsbereich auf. Zwei aliphatische Protonen sind im Bereich um 2,7 ppm identifizierbar, weitere aliphatische Signale (3 Protonen) überlagern mit den $-CH_xOH/-CH_xOR$-Signalen. Ein Teil der Signale tritt verdoppelt bzw. verbreitert auf. Einzelne Integrale entsprechen daher nominell nur einem Isomer, während andere für beide Isomere gemeinsam integriert werden.

<u>$^{13}$C-NMR</u>

Das $^{13}$C-NMR-Spektrum der Substanz zeigt für die Mehrheit der Kohlenstoffatome eine Signalverdopplung. Das Verhältnis der beiden Isomere schwankt abhängig vom verwendeten Lösungsmittel leicht. Es treten elf Signale im Bereich zwischen 25 bis 90 ppm für aliphatische Kohlenstoffatome sowie 24 Signalgruppen im für olefinische Kohlenstoffatome typischen Bereich zwischen 90 und 165 ppm auf. Ein zusätzliches Signal bei 205 ppm wird vom Lösungsmittel überlagert, kann allerdings in d6-DMSO eindeutig identifiziert werden. Aufgrund dieser Überlagerung wurden alle weiteren 2D-Experimente in d6-DMSO durchgeführt.

Die Messung des DEPT(Distortionless Enhancement by Polarization Transfer)-Spektrums erfolgte in d6-Aceton. Das DEPT-Spektrum weist neben drei Methylengruppen eine aliphatische Methingruppe sowie sieben -CHOH-/-CHOR-Signale im Bereich zwischen 65 bis 85 ppm auf. Acht zusätzliche Signale zwischen 95 bis 125 ppm sind olefinischen CH-Gruppen zuzuordnen. Durch Abgleich mit den $^{13}$C-NMR-Signalen kann auf das Vorliegen von 17 quartären Kohlenstoffatomen geschlossen werden.

<u>H/H-Korrelation (COSY(Correlation Spectroscopy))</u>

Das Korrelationsspektrum liefert nur wenige auswertbare Signale, da ein Großteil der Wechselwirkungen im stark überlagerten Bereich von 3,1 bis 3,5 ppm zusammenfallen. Die Signalgruppe H2" bis H4" ist im Bereich von 4,0 bis 4,4 ppm identifizierbar, ebenso ist die Korrelation der Alkylkette H7 (2,7 ppm) bis H8 (3,3 ppm) eindeutig. Ausgehend von H1' bei 4,7 / 4,8 ppm ist nur eine Korrelation in den Bereich bei 3,1 bis 3,5 identifizierbar.

<u>C/H-Korrelationsspektren (HMQC(Heteronuclear Multiple Quantum Correlation), HMBC(Heteronuclear Multiple Bond Correlation))</u>

Aus den CH-long-range-Korrelationsspektren (HMBC) sind die zu quartären Kohlenstoffatomen benachbarten Protonen sowie auch die Verknüpfung der einzelnen Strukturelemente ableitbar.

Über die Zuordnung der direkten Korrelationen kann ebenfalls geprüft werden, ob die Strukturzuordnung plausibel ist.

Auswertung der erhaltenen Daten und Abgleich mit Literaturwerten

Das Kopplungsmuster der Protonen im Bereich von 6,8 bis 6,4 ppm weist eindeutig auf zwei sich stark ähnliche aromatische Systeme mit 1,3,4-Substitution hin. Anhand der Kopplungen aus den HMBC-Spektren sowie den chemischen Verschiebungen der beteiligten Kohlenstoffatome (145 / 143 / 131 / 119 / 116 / 115 ppm) kann eine 3,4-dihydroxybenzylische Struktur abgeleitet werden. Als Kontaktstellen ist eine Methylengruppe bei 2,7 ppm (30 ppm) - die C7 entspricht - sowie eine -CHOR-Gruppe bei 4,4 ppm (82,5 ppm) - die C2" zugeordnet wird - identifizierbar. Das Fragment mit C7 lässt sich über die zweite Methylengruppe (C8: 3,26 / 46,0 ppm) bis zum Keton an C9 (205 ppm) eindeutig verlängern. Ausgehend von C9 sind dann aber keine weiterführenden Korrelationen erkennbar.

Das C2" enthaltende Fragment weist eine Verknüpfung zu einer weiteren CHOR-Gruppe (4,1 ppm / 71 ppm) auf.

Die Methingruppe bei 4,3 ppm bzw. 37,5 ppm lässt sich über H/H-Korrelationen dem Signal von C4" zuordnen. Von dort sind weitere Anknüpfungspunkte zu zwei Signalen im Bereich von 103 bis 105 ppm erkennbar die C14 bzw. C10" zugeordnet werden können sowie einer CHOR-Gruppe.

Die Signalgruppe bei 5,7 ppm (2 H, 94 bzw. 96 ppm) zeigt nur kleine meta-Kopplungen (<3 Hz) und kann einem 1,3,5,6-tetrasubstituierten Aromaten mit 3 Sauerstofffunktionen mit weiteren Signalen bei 163 / 161 / 158 und 103 ppm zugeordnet werden. Aufgrund der unterschiedlichen Verschiebung kann eine symmetrische Substitution ausgeschlossen werden.

Die noch nicht zugeordneten sechs $-CH_xOH$ / -CHOR-Gruppen können nicht eindeutig zugeordnet werden, da alle Protonensignale, mehrere direkte und nahezu alle Long-range-Kopplungen in der Signalgruppe um 3,3 ppm zusammenfallen. Aus den chemischen Verschiebungen der Kohlenstoffatome kann jedoch mit guter Sicherheit auf das Vorliegen einer Kohlenhydrat-Seitenkette geschlossen werden.

Die Konnektivität der verbleibenden sechs Signale im Aromatenbereich kann nicht eindeutig geklärt werden, da es sich um sechs quartäre Signale handelt. Diese sind auch über HMBC-Spektren nicht auflösbar.

Damit ergaben sich die folgenden Strukturelemente **A** bis **D** gemäß Figur 4.

Bei näherer Betrachtung lassen sich aus diesen Strukturelementen zwei Naturstoffe konstruieren. So ergibt die Verknüpfung von i mit iii ein in C4"-Position substituiertes Catechin / Epicatechin, während durch Verknüpfung von iv mit vii und von v mit viii ein in Position C14 substituiertes Aspalathinderivat gebildet wird. Beide Substanzen sind aus Rotbuschtee bekannt. Die verbleibenden offenen Verknüpfungen C14 und C4" waren bereits im Verlauf der Elementzuordnung als verknüpft identifiziert worden.

Ein Vergleich der $^{13}$C-NMR-Daten von Aspalathin und einem C4"-substituierten Catechin mit der Verbindung gemäß Formel I (Figur 5) liefert eine gute Übereinstimmung der korrespondierenden Werte. Das Vorliegen von konformativen Isomeren (Atropisomere, Rotationsisomere) kann durch die Behinderung der freien Rotation um die Bindung C14/C4" gut erklärt werden.

Auf eine Gegenüberstellung der $^{1}$H-NMR-Daten wird verzichtet, da durch Signalüberlagerung und Signalverdopplung - die durch das Vorliegen von Rotationsisomeren bedingt ist - ein sehr komplexes Spektrum für die Verbindung der Formel I erhalten wird.

Da die Substanz bislang nicht in der Literatur beschrieben ist, wurde mittels eines Simulationsprogramms (ACD/C+H NMR Predictor V.10.02) die Plausibilität der Zuordnung überprüft. Die Übereinstimmung war bei Vergleich der $^{13}$C-NMR-Daten sehr gut.

Die Zuordnung der drei unbekannten Stereozentren sowie des Kohlenhydrats ist nicht eindeutig möglich, sie erfolgt auf Basis der Strukturanalogie und wird als catchinoide all trans-Konfiguration und als $\beta$-gluco-Konfiguration zugeordnet.

**Massenspektrum**

Instrument: Micromass Quattro micro API (Fa. Waters); LC-MS
Methode: Electrospray Ionisierung (ESI) / Sekundärionisierung EI

*Ergebnis*

Im ESI-Spektrum tritt ein Molekülradikalkation bei m/z = 741 $[M + H]^+$ sowie ein Clusterion bei m/z = 763 $[M + Na]^+$ auf. Weitere Fragmente treten bei m/z = 453 [M - Catechin]$^+$ und 289 [M - Catechin - Glycosid] auf. Der Substanz ist eine Masse von 740 zuzuordnen.

**Um-Spektrum**

Die Aufnahme des UV-Spektrums der Substanz erfolgte im Rahmen der Prüfung auf chromatographische Reinheit mit HPLC und Dioden-Array-Detektion (DAD) (Trennsystem 1).

*Ergebnis*

Das UV-Spektrum zeigt ein Absorptionsmaximum bei 285 nm und eine Schulter bei 228 nm.

**Beispiel 3 -** Tele-Stereo-EEG-Untersuchungen

Die Veränderungen der EEG(Elektroenzephalografe)-Frequenzen wurde nach Gabe von Salzlösung (Kontrolle) bzw. der Verbindung der Formel I oral (3 oder 6 mg/kg Körpergewicht bestimmt.

Die Untersuchungen wurden analog der durch W. Dimpfel beschriebenen Methode (Dimpfel, W., Preclinical data base of pharmaco-specific rat EEG fingerprints (Tele-Stereo-EEG). Eur. J. Med. Res. (2003) 8: 199-207) folgendermaßen durchgeführt:

Sechs männlichen erwachsenen Fischer-344 Ratten (Tag-Nacht konvertiert) wurden im Alter von 6 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippokampus, Striatum und Formatio Reticularis abgeleiteten Feldpotentiale. Die Signale wurden auf einem Computer System (Software "EEG-Analyse", Betriebssystem OS Science, Laborrechner "LabTeam" der Firma MediSyst, Linden, DE) in Echtzeit einer Fast-FourierTransformation unterworfen und die Leistungsdichtespektren jeweils über 60 Minuten gemittelt. Die Unterteilung der Spektren in 6 verschiedene Frequenzbereiche erlaubte die Erfassung pharmako-spezifischer Veränderungen in Bezug auf die jeweils vor Applikation gemessenen Vorwerte innerhalb dieser Frequenzbänder.

Zum Applikationsprotokoll der Substanzen: die Substanzen wurden oral 45 Minuten nach Beginn der Messungen (Vorwert) appliziert. Fünf Minuten später wurden die Messungen wieder gestartet, mindestens über die nächsten 5 Stunden kontinuierlich analysiert und in 60-minütigen Perioden zusammengefasst. Die Testsubstanz wurde in einer Dosierung von 3 und 6 mg/kg (Verbindung der Formel I). appliziert. Die experimentelle Serie wurde mit der Gabe von Salzlösung (Kontrolle), die zu keiner auffälligen Änderung führte, begonnen (Fig. 6).

Der statistische Vergleich der Versuche zu den Ergebnissen, die nach Gabe von Salzlösung gemessen wurde, erfolgte mit Hilfe einer multivariaten Analyse nach Ahrens und Läuter (siehe H. Ahrens, J. Läuter "Mehrdimensionale Varianzanalyse" (1974), Akademie Verlag, Berlin) auf der Basis der Veränderungen innerhalb der einzelnen Frequenzbänder in allen Hirnregionen als Variablen.

Die Verabreichung von Salzlösung führte kaum zu Veränderungen der elektrischen Aktivität ($\mu V^2/\Omega$) im Vergleich zu den Vorphasenwerten (Fig. 6).

Die Verabreichung der Verbindung gemäß Formel I führte zu stabilen Veränderungen der Leistungsdichte in allen Hirngebieten, vor allem während der zweiten und dritten Stunde nach Applikation (siehe Fig. 7 und 8), wobei die Veränderungen sich von denen nach Gabe von Salzlösung trotz der geringen Tierzahl signifikant unterschieden. Bei etwa äquimolarer Gabe von Aspalathin, Catechin oder Epicatechin kann die Wirkung auf Grund der einzigartigen Struktur nur von der Verbindung gemäß Formel I, nicht jedoch durch Teile des Moleküls erreicht werden (Fig. 9). Dies ist auf die besondere Bindungs-Struktur des neu entdeckten Moleküls zurückzuführen. Eine Steigerung der Motilität wurde lediglich in Gegenwart von (-)-Epicatechin beobachtet (Fig. 11) nicht jedoch in Gegenwart der Verbindung gemäß Formel I. Dies betont die eigenständige Wirkung der Verbindung gemäß Formel I.

Die orale Verabreichung der Verbindung gemäß Formel I als Einzeldosis führt zu Veränderungen der elektrischen Hirnaktivität bei den Testtieren, die denen nach Gabe von Metanicotin, Galantamin, Tacrin, L-DOPA, oder Nefopam entsprach. Dieses Muster korreliert in auffälliger Weise mit den Mustern, die für Medikamente, wie sie bereits für die Behandlung von Demenz, Morbus Parkinson und Schmerzzuständen üblicherweise verwendet werden (siehe Fig. 10), nicht jedoch mit Mustern, die bei Medikamenten für andere Indikationen auftreten (Fig. 11).

Die beobachteten Frequenzänderungen wurden mittels einer Diskriminanzanalyse mit den Ergebnissen von Medikamenten zur Behandlung psychiatrischen Erkrankungen des Gehirns wie auch mit Medikamenten für andere Indikationen verglichen. Beispielhaft können die Wirkstoffe: Galantamin, Nefopam, LSD, Metanicotin, Koffein, Tacrin,

Acetylsalicylsäure, Methadon, Metamizol, Fentanyl, Fluvoxamin, Chlorpromazin, Haloperidol, Methohexital, Meprobamat, Midazolam, Valproinsäure und Carbamazepin aufgeführt werden.

Die Ergebnisse einer Diskriminanzanalyse auf der Basis von vier Hirnregionen und 6 Frequenzbändern (24 Variable) zeigten, dass sich die Wirkung der Verbindung gemäß Formel I in die Nachbarschaft von Galantamin, Tacrin sowie Nefopam einordnet.

**Beispiel 4 -** Messverfahren zur Bestimmung des Gehalts an Verbindung gemäß Formel I

Der Gehalt der Verbindung nach Formel I in Rotbusch und Zubereitungen aus Rotbusch (Tee, Extrakt, Tabletten) wird mittels HPLC/DAD nach der Methode des externen Standards bestimmt. Zur Gehaltsbestimmung wird die Substanz der Verbindung gemäß Formel I als externer Standard verwendet. Die Auswertung erfolgt bei 280 nm Detektionswellenlänge. Um Oxidationsvorgänge in der Analyselösung zu verhindern, wird den Proben Askorbinsäure zugesetzt.
Das bevorzugt verwendete HPLC-Gerät ist Acquity UPLC/Alliance 2695; Detektor: DAD, 200 bis 400 nm; Säule: Reprosil-Pur ODS-3, 125 x 3 mm, 3 µm, Fa. Dr. Maisch; Säulentemperatur: 60°C.

Eluent: Eluent A: Wasser/Ameisensäure 100/0,2 (V/V);
Eluent B: Acetonitril/Methanol/Wasser/Ameisensäure 50/25/25/0,2 (V/V/V/V).

Injektionsvolumen: 20 µL.
Laufzeit: 95 Min.
Retentionszeit der Verbindung gemäß Formel I: 39,5 Min.

Tabelle 1 (Gradient):

| Zeit (Min.) | Fluss (ml/Min.) | Eluent A % | Eluent B % | Gradientensteigung |
|---|---|---|---|---|
| 0 | 0,3 | 100 | 0 | |
| 40 | 0,3 | 70 | 30 | linear |
| 60 | 0,5 | 20 | 80 | linear |
| 80 | 0,5 | 20 | 80 | linear |
| 81 | 0,3 | 100 | 0 | |
| 95 | 0,3 | 100 | 0 | |

Als Standardlösung wird verwendet:

1 mg der Verbindung gemäß Formel I, genau gewogen, und ca. 20 mg Askorbinsäure werden mit 2 ml Methanol gelöst und mit Wasser zu 20,00 ml aufgefüllt.
Sollkonzentration: 0,05 mg/mL der Verbindung gemäß Formel I und 1 mg/mL Askorbinsäure.
Als Analysenlösung wird verwendet:

Arzneimittelformulierung:

Die zu untersuchende Probe, beispielsweise eine Tablette, wird in einer Pulvermühle pulverisiert und über einem Sieb mit Maschenweite 250 µm gesiebt.
Ca. 0,5 g pulverisierte Probe werden zusammen mit ca. 50 mg Askorbinsäure genau in einen 50 mL Messkolben eingewogen, mit 10 mL Methanol 40°C versetzt und für 10 Min. im Ultraschallbad bei 40°C extrahiert. Anschließend wird mit Wasser bis zur Marke aufgefüllt, kräftig geschüttelt und erneut für 10 Min. im Ultraschallbad bei 40°C extrahiert. Nach dem Erkalten wird gegebenenfalls mit Wasser bis zur Marke aufgefüllt und die
Lösung für 5 Min. bei 9300 g zentrifugiert. Der Überstand wird über einen 0,45 µm Membranfilter direkt in ein Braunglasfläschchen für den automatischen Probengeber der HPLC-Anlage filtriert.

Trockenextrakt Auszugsmittel Wasser:

Ca. 125 mg Rotbuschextrakt werden zusammen mit ca. 25 mg Askorbinsäure in einen 25 mL Messkolben gewogen, mit ca. 22 mL Wasser versetzt, kräftig geschüttelt, gegebenenfalls im Ultraschallbad behandelt und mit Wasser bis zur Marke aufgefüllt.

Trockenextrakt Auszugsmittel nicht Wasser:

Ca. 125 mg Rotbuschextrakt werden zusammen mit ca. 25 mg Askorbinsäure in einen 25 mL Messkolben gewogen, mit ca. 2,5 mL Methanol versetzt und für 10 Min. im Ultraschallbad behandelt. Anschließend wird mit Wasser

bis zur Marke aufgefüllt, kräftig geschüttelt und erneut für 10 Min. im Ultraschallbad behandelt.

Auswertung:

Standardlösung und Analyselösung werden direkt hintereinander unter den selben Bedingungen chromatographiert. Die UV-Spektren der Referenzsubstanz werden mit der zur selben Retentionszeit im Analysenchromatogramm detektierten Substanz verglichen und bei Übereinstimmung der Peak als Verbindung gemäß Formel I nach folgender Berechnungsformel berechnet:

$$\text{Gehalt [\%]} = \frac{\text{Peakfläche Analyse} \cdot \text{Verdünnung Analysenlösung (mL)} \cdot \text{Einwaage Standard (mg)} \cdot 100}{\text{Peakfläche Standard} \cdot \text{Verdünnung Standardlösung (mL)} \cdot \text{Einwaage Analyse (mg)}}$$

**Beispiel 5A -** Formulierungen von Monopräparaten der Verbindung gemäß Formel I

Der Ausdruck "Verbindung I" in den Beispielen bezeichnet alle Verbindungen gemäß Formel I sowie deren pharmazeutisch verträgliche Salze, Derivate und Ester.

Filmtablette:

| | |
|---|---|
| Verbindung I | 50 mg |
| Askorbinsäure | 60 mg |
| Sorbitpulver (Karion instant) | 120 mg |
| Aerosil (hochdisperses Siliciumdioxid) | 3 mg |
| Tablettierhilfsmittel K | 2,8 mg |
| | 235,8 mg |

Film:

| | |
|---|---|
| Schellack | 3,5 mg |
| Ethanol (Lösungsmittel für Schellack) | 97% ca. 70 mg |

Hartgelatinekapsel:

Kapselfüllmasse:

| | |
|---|---|
| Verbindung I | 20 mg |
| Askorbinsäure | 60 mg |
| Maltodextrin | 120 mg |
| Aerosil (hochdisperses Siliciumdioxid) | 2 mg |
| Magnesiumstearat | 1,5 mg |

**Beispiel 5B -** Formulierungen von Monopräparaten mit Extrakt enthaltend die Verbindung gemäß Formel I

Filmtablette:

| | |
|---|---|
| Extrakt (mit Verbindung gemäß Formel I) | (1%) 150 mg |
| Askorbinsäure | 60 mg |
| Sorbitpulver (Karion instant) | 80 mg |
| Aerosil (hochdisperses Siliciumdioxid) | 3 mg |
| Magnesiumstearat | 2,5 mg |
| | 295,5 mg |

Film:

| | |
|---|---|
| Schellack | 4,2 mg |
| Ethanol (Lösungsmittel für Schellack) | 97% ca. 84 mg |

Hartgelatinekapsel:

Kapselfüllmasse:

| | |
|---|---|
| Extrakt (mit Verbindung gemäß Formel I) (1%) | 150 mg |
| Askorbinsäure | 40 mg |
| Maltodextrin | 20 mg |
| Aerosil (hochdisperses Siliciumdioxid) | 1 mg |
| Magnesiumstearat | 1,5 mg |
| | 212,5 mg |

**Beispiel 6 -** Extrakt mit erhöhtem Gehalt an Substanz der Formel I und erhöhtem Gesamflavonoidgehalt

a) Extraktion

Als Ausgangsdroge wurden besonders schnell und schonend getrocknete Blätter und Zweigspitzen von Aspalathus linearis (nicht fermentiert) = "grüner" Rotbusch mit einem möglichst geringen Restfeuchte-Gehalt (<5 %) eingesetzt.

Der Extrakt wurde gewonnen durch verschiedene Extraktionsmittel: Methanol 20 % oder Ethanol 30 % sowie 50 % Ethanol in Wasser. Alternativ kann zuerst der reine Alkohol mit der Droge versetzt werden und nach einer Einweichphase von mind. 30 Minuten die entsprechende Wassermenge zugegeben werden.

Diese Extraktionen sind optimiert auf die möglichst vollständige Extraktion von Verbindungen der Formel I und gleichzeitig die Gesamt-Flavonoide.

Der Extrakt unterscheidet sich von den bisher handelsüblichen Extrakten für die innerliche Anwendung in Tee-Getränken (rein wässrige Extraktion) durch höhere Gehalte an Gesamtflavonoiden; von dem Extrakt für die äußerliche Anwendung in der Kosmetik (80 % ethanolisch) durch das Verhältnis der Flavonoidgruppen zueinander. Der Ethanol-80 %-Extrakt wurde auf einen möglichst hohen Aspalathin-Gehalt optimiert. Das Verhältnis der 3 Flavonoidgruppen (Aspalathin-Artige, Rutosid-Artige und Vitexin-Artige = C-Glycoside) wird durch die relativ lipophile Extraktion gegenüber dem Ausgangszustand in der Droge verändert. Erkennbar wird dies besonders deutlich am Verhältnis der Vitexin-artigen zu den Rutosid-artigen Flavonoiden.

b) Messverfahren zur analytischen Bestimmung der Komponenten aus den Rohextrakten

Die Ascorbinsäure sowie die verwendeten Lösungsmittel wurden von der Firma Roth (Karlsruhe, Deutschland) bezogen und waren von analytischer Qualität. Die Droge wurde von Firma Rooibos Ltd., Clan William, Südafrika, bezogen.

Die Standards Orientin, Homoorientin und Vitexin sind käuflich erwerbbar, z.B. von der Firma Extrasynthese (Genay, Frankreich) oder von der Firma Roth (Karlsruhe, Deutschland).

Als Spritzenfilter fanden Rotilabo Spritzenfilter 13 mm (0.45 μm, PVDF) von Roth (Karlsruhe, Deutschland) Verwendung.

Für die HPLC-Messung wurde das Gerät HP 1090 Series II Liquid Chromatograph mit Dioden-Array Detector E-3014 (Hewlett Packard) mit der Software ChemStation for LC 3D Rev. A.10.02 (Agilent Technologies) und der Säule Reprosil-Pur ODS-3, 125x3 mm, 3 μm (Firma Dr. Maisch, Ammerbuch, Deutschland) bei einer Säulentemperatur von 60°C eingesetzt.

Zum Einsatz kam das Fließmittel A: Wasser/Ameisensäure 100/0.2 (V/V) und Fließmittel B: Acetonitril/Methanol/Wasser/Ameisensäure 50/25/25/0.2 (V/V), wobei folgende Bedingungen gewählt wurden:

Gradient:

Tabelle 2

| Zeit [min] | Fluss [mL/min] | FM A [%] | FM B [%] | Gradient |
|---|---|---|---|---|
| 0 | 0.3 | 100 | 0 | |
| 40 | 0.3 | 70 | 30 | Linear |
| 60 | 0.5 | 20 | 80 | Linear |
| 80 | 0.5 | 20 | 80 | Linear |
| 81 | 0.3 | 100 | 0 | |
| 95 | 0.3 | 100 | 0 | |
| Injektionsvolumen: 20 μL; Laufzeit: 95 min | | | | |

1.0 mg Standardsubstanz, genau gewogen, und ca. 20 mg Ascorbinsäure werden in 2.0 mL Methanol gelöst und mit Wasser zu 20.0 mL aufgefüllt. Folgende Standards wurden verwendet: Homoorientin, Orientin, Vitexin, Rutosid, Isoquercitrin, Ferulasäure.

Als Standardlösung von Verbindungen der Formel I wurde als Arbeitsstandard eine Lösung des Extraktes G110907SA in Methanol verwendet. Hierzu wurden 125 mg Extrakt G110907SA zusammen mit ca. 25 mg Ascorbinsäure in einen 25 mL Messkolben gewogen und mit ca. 22 mL Wasser versetzt. Nach kräftigem Schütteln und einer Behandlung im Ultraschallbad wird mit Wasser bis zur Marke aufgefüllt. Der Gehalt an Verbindungen der Formel I in diesem Extrakt beträgt 0.95 % und wurde zur Gehaltsbestimmung in den anderen Extrakten verwendet.

c) Messungen

6.c)1) Extraktion : Methanol-20%-Extrakte

0.5 g Extrakt (genau eingewogen) wird zusammen mit ca. 50 mg Ascorbinsäure in einen 50.0 mL Messkolben eingewogen, mit 10 mL Methanol (40 °C) versetzt und für 10 min im Ultraschallbad bei 40°C extrahiert. Anschließend wird mit Wasser bis zur Marke aufgefüllt, kräftig geschüttelt und erneut für 10 min im Ultraschallbad bei 40°C extrahiert. Nach dem Erkalten wird gegebenenfalls mit Wasser bis zur Marke aufgefüllt und die Lösung für 5 min bei 9300×g zentrifugiert.

Vom Überstand wurden 1 ml genommen, über einen Spritzenfilter in ein Vial (Braunglas) filtriert und per HPLC vermessen.

6.c)2) Verbindung der Formel I

Die Identifizierung der Substanz 1 im Chromatogramm des Extraktes 1 (G110907SA erfolgte über ein zur Verfügung stehendes Vergleichsspektrum sowie über das UV-Vergleichs-Spektrum (Figur 12) sowie ein Online UV-Spektrum.

Der Gehalt an Substanz 1 in verschiedenen Extrakten wurde unter Verwendung des bekannten Gehaltes im Extrakt 1 (G110907SA) (0.95%) anhand folgender Formel berechnet (*F*-Fläche, *V*-Volumen, *m*-Masse, *g*-Gehalt, Ana-Analyse, *St*-Standard):

$$g_{Substanz1}(\%) = \frac{F_{Ana}V_{Ana}}{m_{Ana}relF_{St}}100\%$$

Wobei die relative Fläche *relF* folgendermaßen berechnet wurde:

$$relF = \frac{100F_{St}}{V_{Extrakt}m_{Extrakt}0.95} = 12.52 \pm 0.04$$

6.c)3) Flavonoide der Substanz A-Gruppe

Flavonoide der Substanz A-Gruppe sind durch UV-Maxima bei 287 nm und 228 nm charakterisiert. Alle Peaks, die eine weitgehende Übereinstimmung mit diesem Spektrum zeigen (Figur 13), werden zu dieser Gruppe gezählt und der Gehalt anhand folgender Formel berechnet:

$$g_{FlavonoidegruppeA}(\%) = \frac{F_{Ana-GruppeA}V_{Ana}m_{Rutosid}}{m_{Ana}F_{Rutosid}V_{Rutosid}}k_f 100\%$$

Als Standard wird Rutosid verwendet, der Korrekturfaktor $k_f$ beträgt 0.4.

6.c)4) Flavonoide der Rutosid-Gruppe

Flavonoide werden anhand eines Rutosid-Vergleichsspektrums (Figur 14) dieser Gruppe zugeordnet. Der Gehalt wird anhand folgender Formel berechnet:

$$g_{FlavonoideRutosid}(\%) = \frac{F_{Ana-Rutosid}V_{Ana}m_{Rutosid}}{m_{Ana}F_{Rutosid}V_{Rutosid}}100\%$$

6.c)5) Flavonoide der Vitexin-Gruppe

Flavonoide werden anhand eines Vitexin-Vergleichsspektrums dieser Gruppe zugeordnet, zu der auch Orientin und Homoorientin gehören (Figur 15 bis Figur 17). Als Standard wird anstelle des Vitexins Homoorientin verwendet, da Vitexin (und auch Orientin) bei der Herstellung der Standardlösungen Probleme mit der Löslichkeit zeigten. Der Gehalt wird anhand folgender Formel berechnet:

$$g_{FlavonoideVitexin}(\%) = \frac{F_{Ana-Vitexin}V_{Ana}m_{Homoorientin}}{m_{Ana}F_{Homoorientin}V_{Homoorientin}}100\%$$

6d) Ergebnisse

Die bei der Analyse erhaltenen Ergebnisse werden in der nachfolgenden Tabelle 3 zusammengefasst:

Bei dem erfindungsgemäßen Extrakt wurde angestrebt, dass die 3 Flavonoid-Gruppen möglichst in demselben Verhältnis im Extrakt erhalten werden, wie sie in der AusgangsDroge vorliegen (unter Berücksichtigung der natürlichen Schwankungsbreite in der Droge
und durch das Verfahren von Charge zu Charge). Dieses Verhältnis ist dann dem Verhältnis in den üblichen, aus grünem Rotbusch hoher Qualität (geringer Fermentierungsgrad) Tee-Getränken vergleichbar.

**Tabelle 3.** Gehalt an Flavonoiden - Substanz 1, Rutosid-Gruppe, Substanz A und Vitexin-Gruppe bezogen auf die eingesetzte Droge und bezogen auf die unterschiedlich hergestellten Extrakte (ausgehend von der Droge CH G310807SA). Die % Gehalte stellen jeweils Mittelwerte aus mindestens 2 Extraktaufarbeitungen dar. (A) Die Extraktion wurde zunächst 10 Minuten mit dem reinen Alkohol durchgeführt, anschließend wurde Wasser zugegeben, bis der angegebene Alkoholgehalt eingestellt war. (B) Die Extraktion wurde von Anfang bis Ende mit einem Alkohol/Wasser-Gemisch mit dem angegebenen Alkoholgehalt durchgeführt.

| | Gehalt [%] | | | | |
|---|---|---|---|---|---|
| | Substanz I | Rutosid-Gruppe | Substanz A-Gruppe | Vitexin-Gruppe | Droge/Extrakt-verhältnis |
| **Droge (G310807SA)** | **1.09** | **0.56** | **5.81** | **0.87** | |
| Extrakt (A): Methanol 20% | 2.12 (95% 1.9 fach) | 1.85 (230% 3.3 fach) | 13.53 133% 2.3 fach) | 2.52 (190% 2.9 fach) | 3.2:1 |
| Extrakt (A): Ethanol 30% | 2.66 (144% 2.4 fach) | 2.03 (262% 3.6 fach) | 13.51 (132% 2.3 fach) | 2.14 (145% 2.5 fach) | 3.2:1 |
| Extrakt (B): Methanol 20% | 2.57 (136% 2.4 fach) | 2.45 (338% 4.4 fach) | 15.65 (169% 2.7 fach) | 3.14 (261% 3.6 fach) | 3.4:1 |
| Extrakt (B): Ethanol 30% | 3.02 (177% 2.8 fach) | 2.26 (304% 4.0 fach) | 14.51 (149.7% 2.5 fach) | 2.85 (227% 3.3 fach) | 3.2:1 |

In Klammern ist jeweils eingefügt, um wieviel % bzw. um das wieviel fache das entsprechende Flavonoid im Vergleich zur Droge in dem jeweiligen Extrakt enthalten ist.

**Beispiel 7 -** Weitere Reinigung

Hergestellt wurde ein Extrakt mit einem noch höheren Gehalt an Substanz der Formel I und Gesamt-Flavonoiden.

Der Extrakt wurde zuerst wie in Beispiel 6a) beschrieben hergestellt und dann durch Aufreinigung an einer Sephadex-Säule ähnlich dem 1. Sephadex-Schritt der Gewinnung der Reinsubstanz gemäß Beispiel 1 weiter gereinigt.

Gehalt an Verbindung der Formel I: > 5 %.
Gesamt-Flavonoid-Gehalt (Summe Aspalatin-artige + Rutosid-artige + Vitexin-artige ohne
Verbindung gemäß Formel I: > 35 %
Verhältnis Vitexin-artige (=C-Glycoside) zu Rutosid-artigen: </= 1,6
Gehalt Aspalathin-artige: >25 %.

Die ca. Retentionszeiten (HPLC, vgl. Beispiel 6 Bestimmung der Gehalte) betrugen unter den angegebenen Bedingungen:

Verbindung gemäß Formel I: 45 Min.
Aspalathin: 39 Min.
Rutosid: 42 Min.
Orientin/Homo-Orientin: 38 Min.
Vitexin: 41,5 Min.

## Patentansprüche

**1.** Verbindung der Formel I

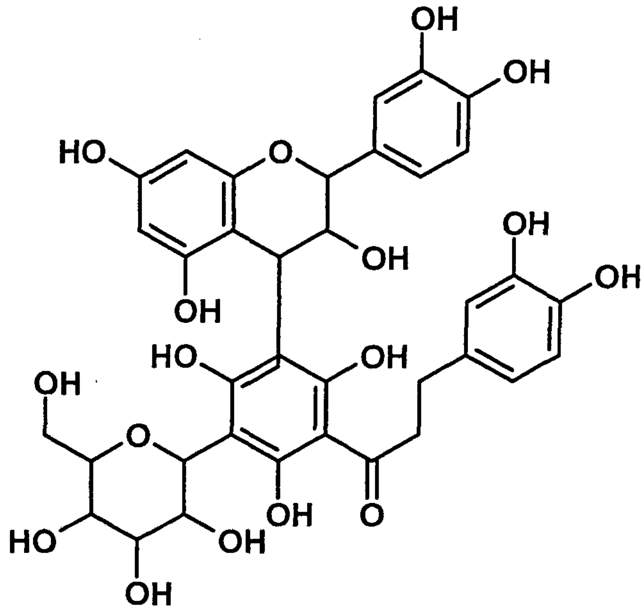

sowie deren pharmazeutisch annehmbare Salze, Derivate, nämlich Kopplungsprodukte der Verbindung gemäß Formel I an Ferulasäure, Chinasäure, Kaffeesäure, Gluconsäure oder Chlorogensäure, und Ester, nämlich Ameisensäure-, Essigsäure-, Propionsäure-, Glutarsäure-, Weinsäure- oder Bernsteinsäureester.

2. Rotbuschextrakt enthaltend mindestens 0,4 Gew % der Verbindung der Formel I gemäß Anspruch 1, **dadurch gekennzeichnet, dass** der Rotbuschextrakt aus unfermentiertem Rotbusch erhalten wird.

3. Rotbuschextrakt gemäß Anspruch 2, **dadurch gekennzeichnet, dass** er einen Gehalt an der Verbindung gemäß Formel I von mindestens 1,0 Gew.-% aufweist.

4. Rotbuschextrakt gemäß Anspruch 3, **dadurch gekennzeichnet, dass** der Gehalt an Verbindung der Formel I wenigstens 2 Gew.-% beträgt.

5. Rotbuschextrakt nach einem der Ansprüche 2 bis 4, **dadurch gekennzeichnet, dass** der Gesamtflavonoidgehalt mindestens 20 Gew.-% beträgt.

6. Verfahren zur Herstellung der Verbindung der Formel I gemäß Anspruch 1 sowie eines Rotbuschextraktes nach einem der Ansprüche 2 bis 5, **dadurch gekennzeichnet, dass**

- getrocknete und zerkleinerte, unfermentierte Rotbusch-Rohware mit einem Extraktionsmittel bestehend aus Alkohol und/oder Wasser für eine vorbestimmte Extraktionsdauer bei einer Temperatur von bis zu 90°C, insbesondere bis zu 60°C extrahiert wird,
- der Extrakt filtriert und anschließend unter reduziertem Druck zur Trockne eingeengt wird,

**dadurch gekennzeichnet, dass** das Extraktionsmittel zu 10 bis 60 % (Vol/Vol) aus Alkohol und der Rest aus Wasser besteht.

7. Verfahren nach Anspruch 6, **dadurch gekennzeichnet, dass** eine weitergehende Aufreinigung durch wenigstens einen chromatographischen Trennungsschritt erfolgt.

8. Verfahren nach Anspruch 7, **dadurch gekennzeichnet, dass** es sich bei dem chromatographischen Trennungsschritt um Größenausschlusschromatographie handelt.

9. Nahrungsergänzungsmittel, **dadurch gekennzeichnet, dass** es mindestens 1 mg der Verbindung der Formel I

gemäß Anspruch 1 pro g Nahrungsergänzungsmittel enthält.

10. Nahrungsergänzungsmittel, **dadurch gekennzeichnet, dass** es Rotbuschextrakt gemäß Anspruch 2 bis 5 in einer Menge von mindestens 10 mg/g Nahrungsergänzungsmittel enthält.

11. Nahrungsergänzungsmittel nach Anspruch 9 oder 10, **dadurch gekennzeichnet, dass** es so zubereitet ist, dass es wenigstens 1 mg der Verbindung der Formel I gemäß Anspruch 1, insbesondere wenigstens 5 mg der Verbindung der Formel I gemäß Anspruch 1 enthält, bezogen auf die Tagesdosis.

12. Arzneimittel, **dadurch gekennzeichnet, dass** es die Verbindung der Formel I gemäß Anspruch 1 oder einen Rotbuschextrakt gemäß einem der Ansprüche 2 bis 5 enthält.

13. Verwendung von Verbindungen gemäß Anspruch 1 oder eines Rotbuschextrakts gemäß einem der Ansprüche 2 bis 5 zur Herstellung eines Medikaments zur Vorbeugung und/oder Behandlung von neurologischen und psychiatrischen Störungen des zentralen Nervensystems.

14. Verwendung gemäß Anspruch 13, worin die neurologischen und psychiatrischen Störungen des zentralen Nervensystems Demenzen, Morbus Parkinson, Depressionen und Schmerzzustände sind.

15. Verwendung gemäß Anspruch 14, worin die Störung des zentralen Nervensystems der Morbus Alzheimer oder Altersdemenz ist.

**Claims**

1. Compound of the formula I

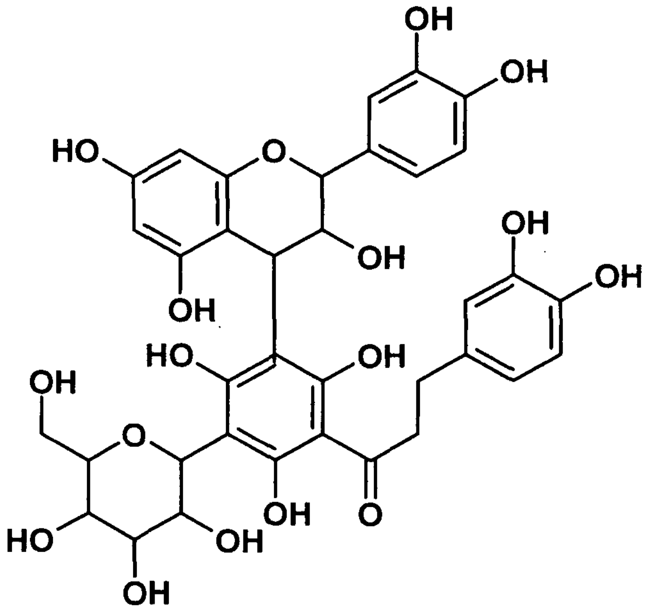

and the pharmaceutically acceptable salts, derivatives, namely coupling products of the compound according to formula I with ferulic acid, quinic acid, caffeic acid, gluconic acid or chlorogenic acid, and esters, namely formic acid, acetic acid, propionic acid, glutaric acid, tartaric acid and succinic acid esters, thereof.

2. Rooibos extract containing at least 0.4 % by weight of the compound of formula I according to Claim 1, **characterized in that** the rooibos extract is obtained from unfermented rooibos.

3. Rooibos extract according to Claim 2, **characterized in that** it has a content of the compound according to formula I of at least 1.0 % by weight.

**4.** Rooibos extract according to Claim 3, **characterized in that** the content of compound of the formula I is at least 2% by weight.

**5.** Rooibus extract according to any of Claims 2 to 4, **characterized in that** the total flavonoid content is at least 20% by weight.

**6.** Process for the preparation of the compound of the formula I according to Claim 1 and of a rooibos extract according to any of Claims 2 to 5, **characterized in that**

- dried and comminuted, unfermented raw rooibos material is extracted with an extracting agent consisting of alcohol and/or water for a predetermined duration of extraction at a temperature of up to 90°C, in particular up to 60°C,
- the extract is filtered and then evaporated to dryness under reduced pressure,

**characterized in that** the extracting agent consists of 10 to 60 % (vol/vol) of alcohol and water as the remainder.

**7.** Process according to Claim 6, **characterized in that** more substantial purification is effected by at least one chromatographic separation step.

**8.** Process according to Claim 7, **characterized in that** the chromatographic separation step is size exclusion chromatography.

**9.** Food supplement, **characterized in that** it contains at least 1 mg of the compound of the formula I according to Claim 1 per g of food supplement.

**10.** Food supplement, **characterized in that** it contains rooibos extract according to Claims 2 to 5 in an amount of at least 10 mg/g of food supplement.

**11.** Food supplement according to Claim 9 or 10, **characterized in that** it is prepared in such a way that it contains at least 1 mg of the compound of the formula I according to Claim 1, in particular at least 5 mg of the compound of the formula I according to Claim 1, based on the daily dose.

**12.** Medicament, **characterized in that** it contains the compound of the formula I according to Claim 1 or a rooibos extract according to any of Claims 2 to 5.

**13.** Use of compounds according to Claim 1 or of a rooibos extract according to any of Claims 2 to 5 for the preparation of a medicament for preventing and/or treating neurological and psychiatric disorders of the central nervous system.

**14.** Use according to Claim 13, wherein the neurological and psychiatric disorders of the central nervous system are dementias, Parkinson's disease, depression and pain.

**15.** Use according to Claim 14, wherein the disorder of the central nervous system is Alzheimer's disease or senile dementia.

## Revendications

**1.** Composé de formule I

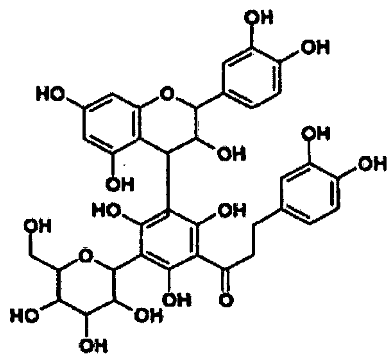

ainsi que ses sels pharmaceutiquement acceptables, ses dérivés, en particulier les produits de couplage du composé selon la formule I avec l'acide férulique, l'acide quinique, l'acide caféique, l'acide gluconique ou l'acide chlorogénique, et des esters, en particulier de l'acide formique, de l'acide acétique, de l'acide propionique, de l'acide glutarique, de l'acide tartrique droit, ou de l'ester de l'acide succinique.

2. Extrait de rooibos contenant au moins 0,4 % en poids de composé de formule I selon la revendication 1 **caractérisé en ce que** l'extrait de rooibos est obtenu à partir de rooibos non fermenté.

3. Extrait de rooibos selon la revendication 2 **caractérisé en ce qu'**il présente une teneur d'au moins 1,0 % en poids de composé de formule I.

4. Extrait de rooibos selon la revendication 3 **caractérisé en ce que** la teneur en composé de formule I est d'au minimum 2 % en poids.

5. Extrait de rooibos selon l'une des revendications 2 à 4 **caractérisé en ce que** la quantité totale de flavonoïdes s'élève à au moins 20 % en poids.

6. Procédé pour la fabrication du composé de formule I selon la revendication 1 ainsi que d'un extrait de rooibos selon l'une des revendications 2 à 5, **caractérisé en ce que**

   - de la matière première de rooibos non fermentée, séchée et émincée est extraite avec un produit d'extraction composé d'alcool et/ou d'eau pendant une durée d'extraction prédéterminée à une température jusqu'à 90°C, en particulier jusqu'à 60 C,
   - l'extrait est filtré et, par la suite, réduit à sec sous pression réduite,

   **caractérisé en ce que** le produit d'extraction est composé de 10 à 60 % (volume/volume) d'alcool et le reste se compose d'eau.

7. Procédé selon la revendication 6 **caractérisé en ce qu'**une purification plus poussée, par au moins une étape de séparation chromatographique, a lieu.

8. Procédé selon la revendication 7 **caractérisé en ce qu'**il s'agit d'une chromatographie d'exclusion stérique dans l'étape de séparation chromatographique.

9. Complément alimentaire **caractérisé en ce qu'**il contient au minimum 1 mg de composé de formule I selon la revendication 1 par g de complément alimentaire.

10. Complément alimentaire **caractérisé en ce qu'**il contient de l'extrait de rooibos selon les revendications 2 à 5 à une quantité d'au minimum 10 mg/g de complément alimentaire.

11. Complément alimentaire selon la revendication 9 ou 10 **caractérisé en ce qu'**il est préparé de sorte qu'il contienne au minimum 1 mg de composé de formule I selon la revendication 1, en particulier au moins 5 mg de composé de formule I selon la revendication 1, pour une dose journalière.

12. Produit pharmaceutique **caractérisé en ce qu'**il contient le composé de formule I selon le revendication 1 ou un extrait de rooibos selon l'une des revendications 2 à 5.

13. Emploi de composés selon la revendication 1 ou d'un extrait de rooibos selon l'une des revendications 2 à 5 pour la fabrication d'un médicament pour la prévention et/ou le traitement de désordres neurologiques et psychiatriques du système nerveux central.

14. Emploi selon la revendication 13 où les désordres neurologiques et psychiatriques du système nerveux central sont des démences, la maladie de Parkinson, des dépressions et des syndromes douloureux.

15. Emploi selon la revendication 14 où le désordre du système nerveux central est la maladie d'Alzheimer ou la démence sénile.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102005004438


### In der Beschreibung aufgeführte Nicht-Patentliteratur


- **BRAMATI et al.** *J. Agric. Food Chem.,* 2003, vol. 51, 7472-7474
- **SCHULZ et al.** *Eur. Food Res. Technol.,* 2003, vol. 216, 539-543
- **PLANTEXTRAKT.** the nature network. Plantextrakt GmbH, 09. November 2005
- **HO et al.** *Phytochemistry,* 1980, vol. 19, 476-477
- **MATTHEWIS et al.** *J. Agric. Food. Chem.,* 1997, vol. 45, 1195-1201
- **DIMPFEL, W.** Preclinical data base of pharmaco-specific rat EEG fingerprints (Tele-Stereo-EEG). *Eur. J. Med. Res.,* 2003, vol. 8, 199-207
- **H. AHRENS ; J. LÄUTER.** Mehrdimensionale Varianzanalyse. Akademie Verlag, 1974